# EUROPEAN PATENT APPLICATION

(11) **EP 2 233 148 A1**
(43) Date of publication of application: **29.09.2010**
(21) Application number: 09703521.6
(22) Date of filing: 16.01.2009
(51) Int. Cl.: A61K 38/17, A61K 48/00, A61P 35/00, A61P 39/00

(54) **USES OF PDCD5 POLYPEPTIDE FOR TUMOR CHEMOTHERAPY AND ORGAN PROTECTION**

(30) Priority: 17.01.2008 CN 200810001144
(71) Applicant: Peking University, Beijing 100871 (CN)
(72) Inventor: WANG, Ying, HaiDian District; Beijing 100191 (CN); MA, Dalong, HaiDian District; Beijing 100191 (CN); SONG, Quansheng, HaiDian District; Beijing 100191 (CN); ZHANG, Yingmei, HaiDian District; Beijing 100191 (CN); LOU, Yaxin, Beijing 100191 (CN); KE, Xiaoyan, Beijing 100191 (CN); SHI, Lin, Beijing 100191 (CN); WANG, Yanfang, Beijing 100191 (CN)
(74) Representative: von Kreisler, Alek
(86) International application number: PCT/CN2009/070174
(87) International publication number: WO 2009/092323

(57) **Abstract**

The invention relates to the use of PDCD5 polypeptides or polynucleotides that code the PDCD5 polypeptides in preparation of sensitizers of chemotherapeutic agents for cancers. The invention also relates to the use of PDCD5 polypeptides or polynucleotides that code the PDCD5 polypeptides to protect normal or diseased tissues or organs from damages or further damages, and to pharmaceutical combinations including chemotherapeutic agents and PDCD5 polypeptides or polyneucleotides that code the PDCD5 polypeptides in an amount for effective sensitization. Preferably, the pharmaceutical agents are selected from antibiotic and anti-metabolic agents or any combination thereof.

SEQUENCE LISTING

<110>  Peking University

<120>  Use of PDCD5 polypeptide in chemical therapy of cancer and organ
protection

<130>  OP080922

<150>  CN/200810001144.3
<151>  2008-01-17

<160>  2

<170>  PatentIn version 3.2

<210>  1
<211>  125
<212>  PRT
<213>  Homo sapiens

<400>  1

Met Ala Asp Glu Glu Leu Glu Ala Leu Arg Arg Gln Arg Leu Ala Glu
1               5                   10                  15

Leu Gln Ala Lys His Gly Asp Pro Gly Asp Ala Ala Gln Gln Glu Ala
            20                  25                  30

Lys His Arg Glu Ala Glu Met Arg Asn Ser Ile Leu Ala Gln Val Leu
        35                  40                  45

Asp Gln Ser Ala Arg Ala Arg Leu Ser Asn Leu Ala Leu Val Lys Pro
    50                  55                  60

Glu Lys Thr Lys Ala Val Glu Asn Tyr Leu Ile Gln Met Ala Arg Tyr
65                  70                  75                  80

Gly Gln Leu Ser Glu Lys Val Ser Glu Gln Gly Leu Ile Glu Ile Leu
                85                  90                  95

Lys Lys Val Ser Gln Gln Thr Glu Lys Thr Thr Thr Val Lys Phe Asn
            100                 105                 110

Arg Arg Lys Val Met Asp Ser Asp Glu Asp Asp Asp Tyr
        115                 120                 125

<210>  2
<211>  599
<212>  DNA
<213>  Homo sapiens

<400>  2
aggccgcgct cgcgccgagg ggctgcgaga gtgaccgcgg ctgctccagc gctgacgccg      60

agccatggcg gacgaggagc ttgaggcgct gaggagacag aggctggccg agctgcaggc     120

caaacacggg gatcctggtg atgcggccca acaggaagca aagcacaggg aagcagaaat     180

gagaaacagt atcttagccc aagttctgga tcagtcggcc cgggccaggt taagtaactt     240

agcacttgta aagcctgaaa aaactaaagc agtagagaat taccttatac agatggcaag     300

atatggacaa ctaagtgaga aggtatcaga acaaggttta atagaaatcc ttaaaaaagt 360
aagccaacaa acagaaaaga caacaacagt gaaattcaac agaagaaaag taatggactc 420
tgatgaagat gacgattatt gaactacaag tgctcacaga ctagaactta acggaacaag 480
tctaggacag aagttaagat ctgattattt actttgttta ttgtctatat gccttttaaa 540
aaaataaact tgttatgcaa aataaaaaaa aaaaaaaaaa aaaaaaaaaa aaaaaaaaa 599

## Description

### FIELD OF THE INVENTION

The present invention relates to cancer chemotherapy and organ protection, and, more particularly, to the use of PDCD5 polypeptides and their derivatives in cancer chemotherapy and organ protection.

### BACKGROUND OF THE INVENTION

*Programmed cell death 5* (*PDCD5*), formerly designated as *TF-1 cell apoptosis-related gene-19* (*TFAR19*), is an increased expression gene during the apoptotic process of TF-1 cells induced by cytokine withdrawal and is cloned using the method of cDNA representational difference analysis (cDNA-RDA method) (Liu et al., Biochem. Biophys. Res. Commun. 254, 203-210 (1999)). Studies showed that transient or stable expression of PDCD5 in cell lines may facilitate apoptosis induced by withdrawal of certain triggers such as growth factors and enhance paraptotic cell death induced by TAJ/TROY. Chen et al. reported that the translocation of PDCD5 from cytoplasm to nucleus is an early signal during the process of apoptosis (Chen et al., FEBS Lett. 509, 191-196 (2001)). Rui et al. demonstrated that the introduction of anti-PDCD5 antibody can suppress the process of apoptosis of HeLa cells induced by VP16 (etoposide) ( Rui et al., Life Sci. 71, 1771-1778 (2002)). The reduction of the expression of endogenous PDCD5 by the use of antisense oligonucleotides can relieve apoptosis of Jurkat cells induced by VP16. These results suggest that PDCD5 plays an important role in apoptosis and non-apoptosis programmed cell death.

A plasma membrane, consisting of a lipid bilayer into which proteins and glycoproteins are inserted, is generally impervious to proteins. Some lager proteins termed translocatory proteins, however, can enter the cell by endocytosis. This group of proteins which is widely used in gene therapy studies includes HIV trans-activator protein Tat, *Drosophila* antennapedia, HSV structual protein VP22, and basic fibroblast growth factor (bFGF). These translocatory proteins share several common features: They are released from cells by a pathway distinct from the recognized secretory routes involving a secretion signal; they bind to target cell in a receptor-independent manner; and each of them has a highly basic region that appears to mediate the ability of these proteins to bind to polyanions, such as heparin/heparin sulfate (HS), polysialic acid, and nucleic acids. Deletion analyses of the first two proteins, Tat and *Drosophila* antennapedia, have mapped the apparent intercellular transfer function to the short runs of highly basic residues (Green & Loewenstein, Cell 55, 1179-1188 (1988); Derossi, et al., J. Biol. Chem. 269, 10444-10450 (1994)) , termed protein transduction domains (PTDs), or cell-permeable peptides (CPPs). CPP-cell delivery system which has broad implication prospects in the fields of biotechnology and pharmacy will become an important tool for cell biology research and clinical drug therapy. *In vitro* and *in vivo* studies have shown that CPP, carrying target molecules such as proteins and nucleic acid into a variety of cells, even neurocytes can regulate gene transcription, cellular signal transduction and cell growth, differentiation and apoptosis; also it can access to organs and tissues including central nervous system through blood brain barrier so that it can be used in therapy for diseases of a variety of tissues and organs such as central nervous, cardiovascular, endocrine, digestive, respiratory, urinary, and reproductive system.

The way how macromolecules like proteins and peptides above enter cells has always been the key in the study. The mechanism of endocytosis is complex, encompassing distinct pathways: clathrin-dependent endocytosis which begins with clathrin-coated invaginations on the plasma membrane that then assembles into clathrin-coated vesicles; and clathrin-independent endocytosis which includes endocytosis mediated by lipid raft and caveolae.

Clathrin-mediated endocytosis is one of the major mechanisms of membrane receptor-specific internalization. Clathrin-coated pits are involved in the internalization, subsequent degradation and recycling, nutrient input and synaptic vesicles formation. Lipid raft and caveolae, being similar in their functions, are membrane domains, which are rich in cholesterol and sphingolipid and are resistant to detergents, formed by plasma membrane invaginations, and participate in signal tansduction and intracellular transport of lipid raft related molecules.

A large body of evidence indicates that cell surface heparin sulfate proteoglycan (HSPGs) are internalized through an endocytic pathway that internalizes ligands that binds to their glycosaminoglycan (GAG) chains. Such an entry mechanism has already been described for other HSPGs ligands, including Tat and bFGF. This is also the case for PDCD5, treatment with heparin (a soluble competitor of cell membrane-associated HSPGs) led to impairment of PDCD5 binding and internalization in a dose-dependent manner in cells, and PDCD5 uptake needs surface HSPGs (Wang et al., J. Biol. Chem. 281, 24803-24817 (2006)).

Caveolae is a special type of cell membrane structure that has lipid raft properties, typically flask-shaped membrane invaginations. Caveolin-1 is a marker protein for caveolae. Methyl-β-cyclodextrin (MβCD) is a drug that extracts cholesterol from cell membrane, thus disrupting lipid rafts. PDCD5 internalization which lipid rafts were involved was significantly inhibited in either HEK293 or U937 cells which were treated with MβCD. Fluorescence microscopy analysis indicated that the internalization of FITC labeled recombinant PDCD5 protein colocalizes partially with caveolin-1; triton X-100 resistance test provided further evidence to define that PDCD5-FITC was localized in lipid rafts (Wang et al., J. Biol. Chem. 281, 24803-24817 (2006)).

Today, chemotherapy is still one of the major methods in cancer treatments; however, the application of chemotherapy has been limited due to the toxicity of the chemotherapeutic drugs. Chemosensitizer refers to a type of substances that can improve efficacy of chemical drugs or reduce toxicities to normal tissues and cells under the effective concentration or dosage and have a synergistic anti-tumor effect, exhibiting increased apoptosis of tumor cells. Therefore, the combination of chemotherapeutic drugs and chemosensitizers plays an important role in clinical applications. Previous study showed that recombinant human PDCD5 (rhPDCD5) protein could accelerate apoptosis of 7721 liver cancer cells induced by hydroxy camptothecin (HCPT) (Li et al., J. Beijing Med. Univ., 408-410 , Vol.32, No.5, 2000). Zhang et al. (Chin. J. Clin. Obstet. Gynecol., 286-289, Vol.5, No.4, 2004) found that rhPDCD5 protein also had a sensitized effect on apoptosis of SiHa cervical cancer cells induced by IFNγ *in vitro.* However, there were no any results of PDCD5 protein *in vitro* to be used by one of ordinary skill in the field to determine or represent the functions, effects and uses of the protein *in vivo*. Prior to this invention, there was no any experimental data to prove the sensitization effect of PDCD5 on tumor chemotherapy and the effects of accelerating tumor cells apoptosis induced by chemotherapeutic drugs.

In addition, many types of chemotherapeutic drugs different from camptothecin (CPT) are used in clinical and most of them have toxic side effects. Therefore, it's necessary to develop programs to enhance chemotherapy efficacy and reduce toxicity of chemotherapeutic drugs. The present invention can meet this need.

### SUMMARY OF THE INVENTION

In one aspect, the present invention provides the use of PDCD5 polypeptides or polynucleotides that code the PDCD5 polypeptides in preparation of sensitizers of chemotherapeutic agents for cancers, such as liver cancer, cervical cancer, and leukemia.

In another aspect, the present invention provides the use of PDCD5 polypeptides or polynucleotides that code the PDCD5 polypeptides in the preparation of pharmaceutical agents to protect normal or diseased tissue or organ from damage or further damages. Preferriably, the normal tissue or organ is heart or liver.

In yet another aspect, the present invention provides a pharmaceutical combination. The pharmaceutical combination includes a chemotherapeutic agent, selected from an antibiotic agent, anti-metabolic agent, or any combination thereof, and a PDCD5 polypeptide in an amount of effective sensitization.

The present invention relates also to a drug composition used for treatmet of cancer, and the composition includes a chemotherapeutic agent in an amount for effective treatment, selected from an antibiotic agent, anti-metabolic agent, or any combination thereof, and a PDCD5 polypeptide in an amount for effective sensitization.

The present invention relates also to the use of PDCD5 polypeptides in the preparation of pharmaceutical agents to protect normal or diseased tissue or organ from damages or further damages.

The present invention relates further to a method for treatment of cancers (such as liver cancer, cervical cancer, and leukemia), and the method includes, prior to, after or during the period of applying chemotherapeutic agent to a subject, applying additional PDCD5 polypeptide or polynucleotide that codes the polypeptide in an amount for effective treatment.

In one embodiment, the PDCD5 polypeptides is selected from the following group: (1) polypeptides that has an amino acid sequence as shown in SEQ ID No:1; or (2) polypeptides that has an amino acid sequence containing at least 70%, 75%, 80%, 85%, 90%, 95% or 97% of homology of SEQ ID No: 1, and has substantially the same biological function or activity comparing to that of the polypeptides of (1); or (3) a functional fragment, variant, analog or derivative of the polypeptide of (1) or (2), having substantially the same biological function or activity comparing to that of the polypeptide of (1) or (2).

In another embodiment, the polynucleotide includes: (a) a nucleotide sequence that codes the amino acid sequence as shown in SEQ ID No: 1; (b) a nucleotide sequence that hybrids with that of (a) under strict conditions; or (c) a nucleotide sequence that has at least 70%, 75%, 80%, 85%, 90%, 95% or 97% of consistency comparing to that of the nucleotide sequence as described in (a).

In the present invention, the biological functions or activities of polypeptides as described herein include, but not limited to, promotion of apoptosis, sensitization of chemotherapeutic agents, protection of normal or diseased organs from damages or further damages or any combination thereof.

In the present invention, the chemotherapeutic agents can be selected from any suitable types, preferrably selected from antibiotic agents, antimetabolic agents, or any combination thereof, more preferrably selected from nucleatide synthesis interfering agents, DNA intercalating agente that interfere with RNA transcription, mitochondria effecting agents, or any combination thereof, and most preferrably selected from daunorubicin, idarubicin, doxorubicin, cytarabine, arsenic acids, or any combination thereof.

### BRIEF DESCRIPTION OF THE DRAWINGS

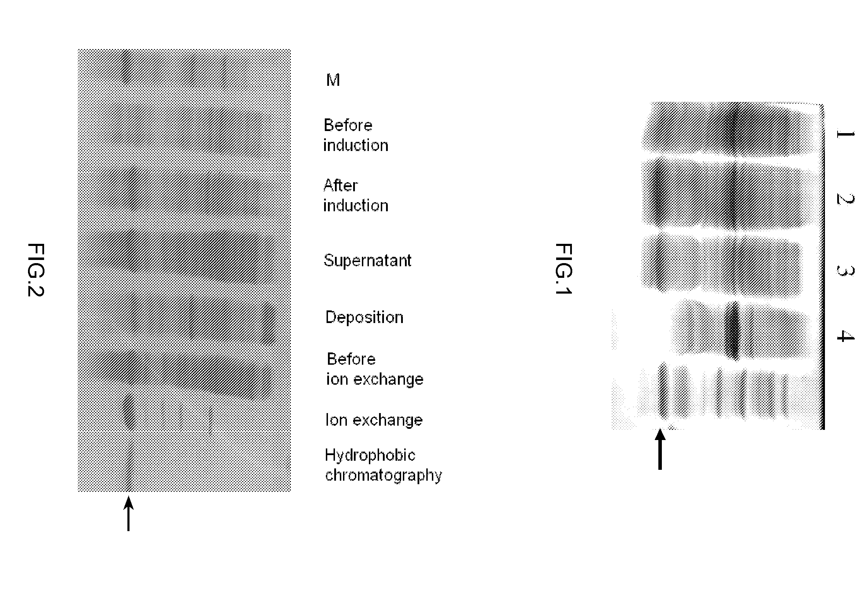

Figure 1 shows the results of expression of rhPDCD5 protein, wherein: Lane 1 is the product before induction, lane 2 is the product after induction, lane 3 is the supernatant after ultrasonic lysis, lane 4 is the deposition of the ultrasonic lysis, and the arrows indicate the position of rhPDCD5 protein.
Figure 2 shows electrophoresis results of samples from steps in purification of rhPDCD5 protein.

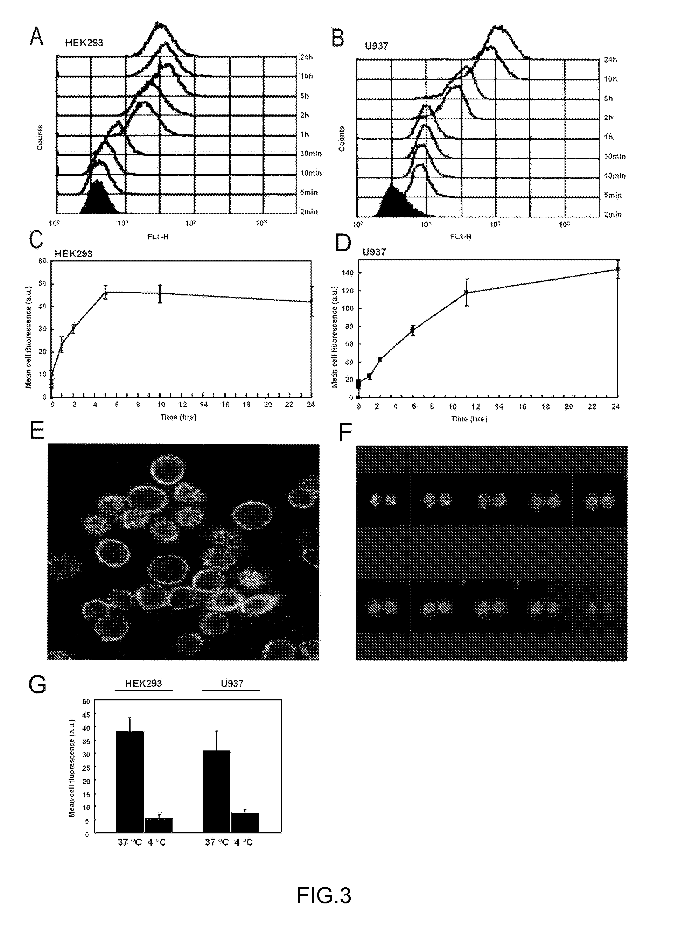

Figure 3 illustrates extracellular PDCD5-FITC internalization dynamics, wherein: A-B shows different time point of HEK293 and U937 cells incubated with 5 µg/mL PDCD5-FITC and fluorescence detected by flow cytometry. Solid peak shows the fluorescence of cells without incubating with recombinant protein; C-D are the average fluorescence values of HEK293 and U937cells incubated with 5 µg/mL PDCD5-FITC after the time point shown in the drawing; E is a image of cells after incubating with 20 µg/mL PDCD5-FITC for 5 hours; F is a series of HEK293 cells' dimension scanning by TCS-SP laser under confocal microscope and the cells were treated with 20 µg/mL PDCD5-FITC for 5 hours; the nucleuses were stained by Hoechst 33342 (red, false color); G reflects the uptake in HEK293 cells and U937 cells incubated with PDCD5-FITC at 4 °C, as shown, the uptake of PDCD5 has been inhibited at 4 °C, and the histogram shows the means of three independent experiments.

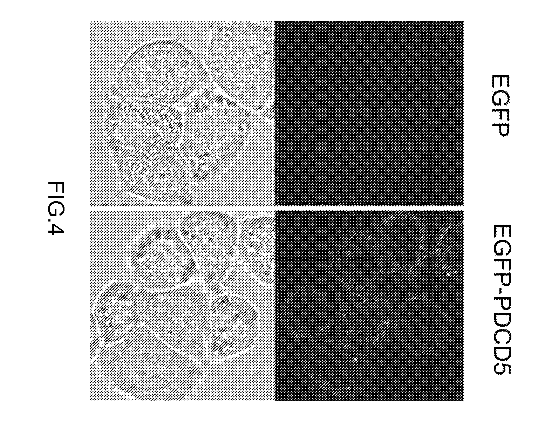

Figure 4 shows the results indicating that PDCD5 can deliver EGFP to cells, wherein: Prokaryotic expression of EGFP protein (A-B) and PDCD5-EGFP fusion protein (C-D) were added respectively to cell culture; PDCD5-EGFP fusion protein can enter into cytoplasm; A and C are fluorescent images; B and D are transmitted light images.

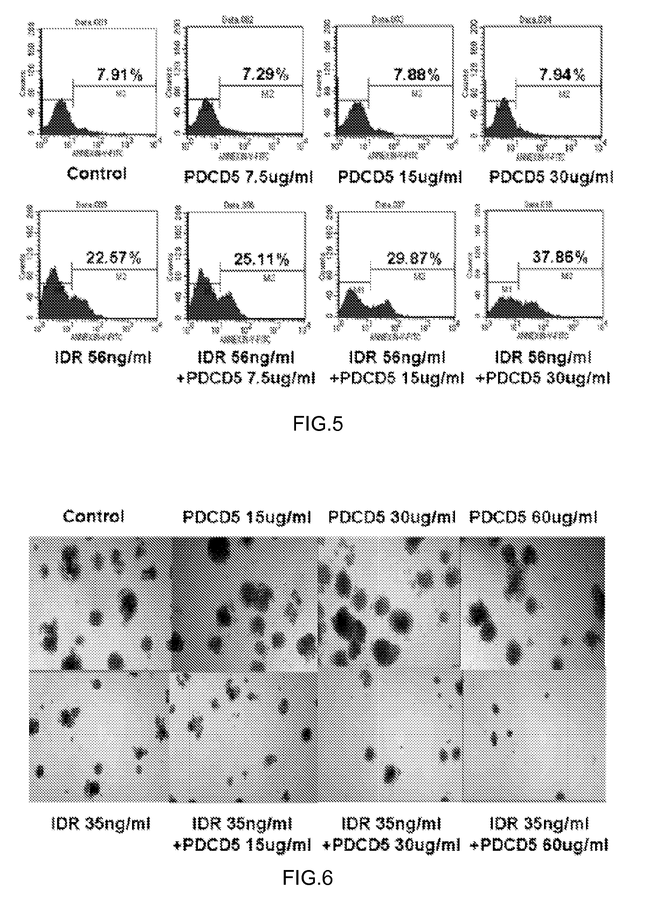

Figure 5 shows the results of FITC/Annexin-V staining, and cell apoptosis in vitro detected by flow cytometry; the results indicate that the apoptosis rate of the group treated with chemotherapeutic drug alone was 22.57%; the rate of the group treated with IDR and PDCD5 reached 37.86% when the concentration of PDCD5 was 30 µg/mL.
Figure 6 shows the results of colony formation assay, indicating that the treatment with both rhPDCD5 and IDR reduced the number of K562 cell colonies in a larger amount as compared to the treatment with IDR alone.

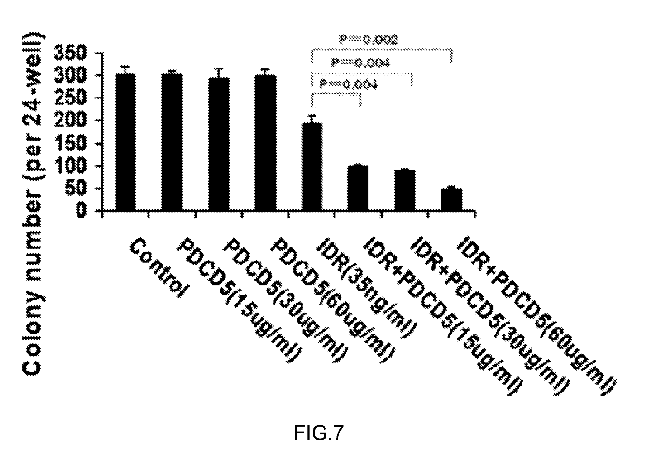

Figure 7 shows statistics of the number of colony formation, which indicates that the treatment with rhPDCD5 in combination of IDR reduced the number of K562 cell colonies in a larger amount as compared to the treatment with IDR alone. Clonogenic inhibition: The rate of colongenic inhibition to K562 cells in the treatment with IDR alone is 35.47%, while the rate of colongenic inhibition to K562 cells in case of the treatment with PDCD5 in combination of IDR can reach up to 81.20%.

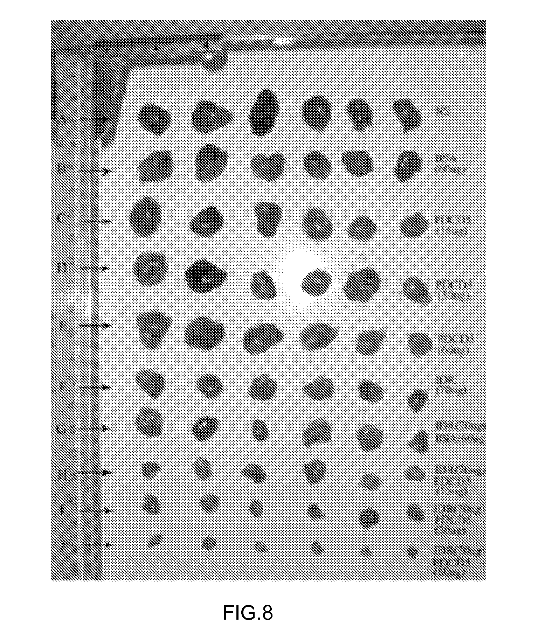

Figure 8 shows the results of local injection of tumor-bearing mice with PDCD5 protein, which indicates that combined treatment with PDCD5 and IDR reduced the tumor volume more significantly as compared with the treatment with IDR alone.

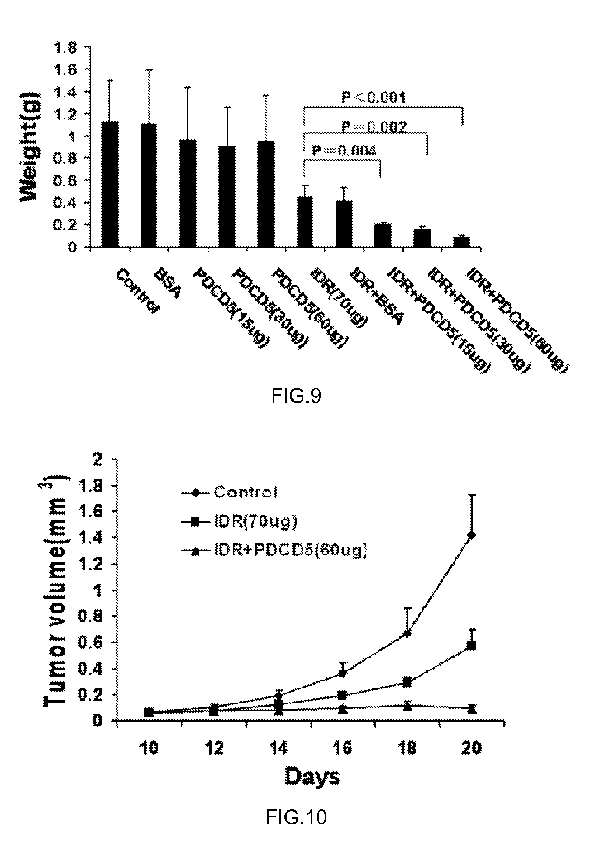

Figure 9 shows the results of local injection of tumor-bearing mice with PDCD5 protein, which indicate that the treatment with PDCD5 in combination of IDR reduced the tumor weights more significantly as compared with the treatment with IDR alone; the tumor weights decreased gradually with the concentration of PDCD5 increased in the treatment with PDCD5 in combination of IDR, while the tumor weights show no significant changes when comparing the treatment with BSA in combination of IDR and the treatment with IDR alone.
Figure 10 shows the results of local injection of tumor-bearing mice with PDCD5 protein, which indicate that the tumor growth was slower in the treatment with PDCD5 in combination of IDR as compared with the treatment with IDR alone.

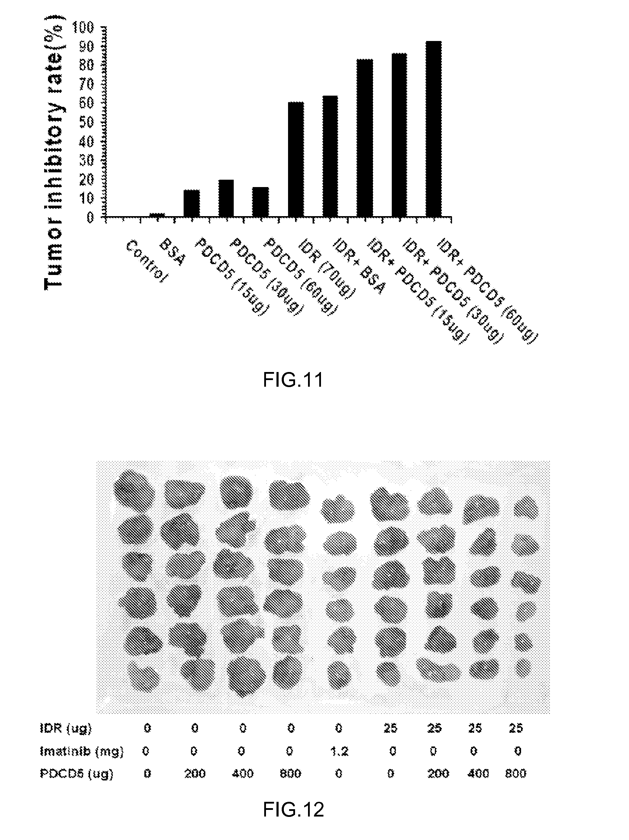

Figure 11 shows the results of local injection of tumor-bearing mice with PDCD5 protein, which indicate that the treatment with PDCD5 in combination of IDR increased the tumor inhibitory rate as compared with the treatment with IDR alone; the tumor inhibitory rate of the treatment with IDR alone was 60.27%, while the rate increased gradually with the concentration of PDCD5 increased, and the rate can reach to 92.02% in the treatment with PDCD5 (60 µg) in combination of IDR.
Figure 12 shows the results under the circumstance that the treatment began when the average tumor volumes reached 140 mm³ while rhPDCD5 protein was administered intraperitoneally. The results indicate that tumor volumes decreased significantly after the treatment with PDCD5 in combination of IDR compared with the treatment of IDR alone, and tumor volumes reduced gradually as PDCD5 concentration increased in case of the combined treatment.

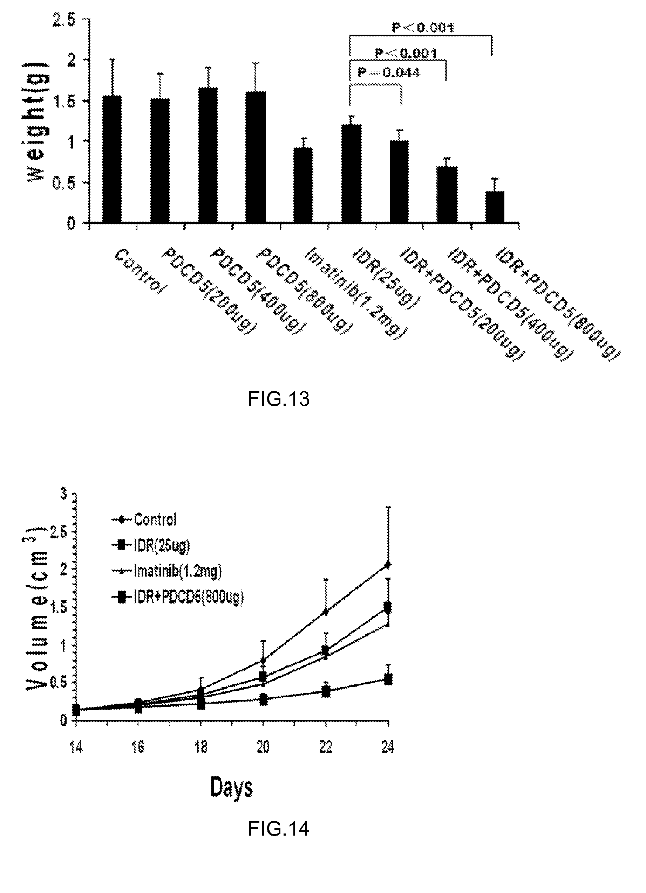

Figure 13 shows the results under the circumstance that the treatment began when the average tumor volumes reached 140 mm³ while rhPDCD5 protein was administered intraperitoneally. The results indicate that tumor weights decreased significantly after treatment of PDCD5 in combination of IDR compared with the treatment with IDR alone, and tumor weights reduced gradually as PDCD5 concentration increased in case of the combined treatment.
Figure 14 shows the results under the circumstance that the treatments began when the average tumor volumes reached 140 mm³ while rhPDCD5 protein was administered intraperitoneally. The results indicate that the tumor growth is slower in the treatment with PDCD5 in combination of IDR as compared with the treatment with IDR alone.

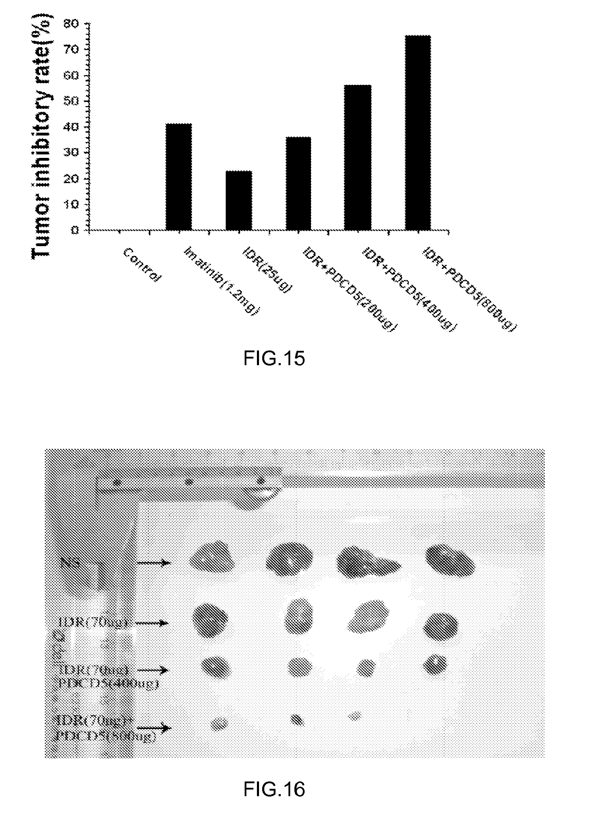

Figure 15 shows the results under the circumstance that the treatments started when the average volume of tumor-bearing mice reached 140 mm³ while rhPDCD5 protein was administered intraperitoneally. The results indicate that IDR combined with PDCD5 significantly suppressed tumor growth compared with IDR alone, the tumor inhibitory rate the treatment with rhPDCD5 (800 µg) and IDR was 75.30%, while the inhibitory rate in the treatment with IDR alone was 35.77%. And the inhibitory rate of the imatinib therapy group was 40.82%. In the treatment with IDR in combination of PDCD5, the inhibitory rate of tumor growth increased gradually with the increase of the concentration of PDCD5.
Figure 16 shows the results under the circumstance that the treatments started when the average volume of tumor-bearing mice reached 57.7 mm³, and rhPDCD5 protein was administered intraperitoneally. The results indicate that IDR combined with PDCD5 significantly decreased the volume of tumor compared with IDR alone, and the volume of tumor decreased gradually with the increase of the concentration of PDCD5 in the treatment with both IDR and PDCD5.

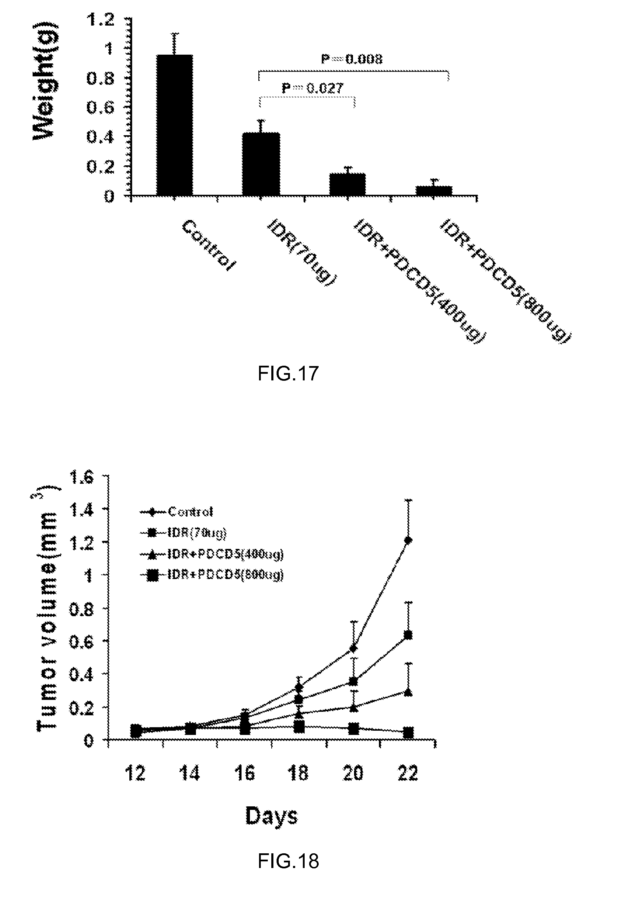

Figure 17 shows the results under the circumstance that the treatments started when the average volume of tumor-bearing mice reached 57.7 mm³, and rhPDCD5 protein was administered intraperitoneally. The results indicate that IDR combined with PDCD5 significantly decreased the weight of tumor compared with IDR alone, and the weight of tumor decreased gradually with the increase of the concentration of PDCD5 in the case of the treatment with both IDR and PDCD5.
Figure 18 shows the results under the circumstance that the treatments started when the average volume of tumor-bearing mice reached 57.7 mm³, and rhPDCD5 protein was administered intraperitoneally. The results indicate that the tumor growth is slower in the treatment with IDR in combination of PDCD5 as compared with the treatment with IDR alone.

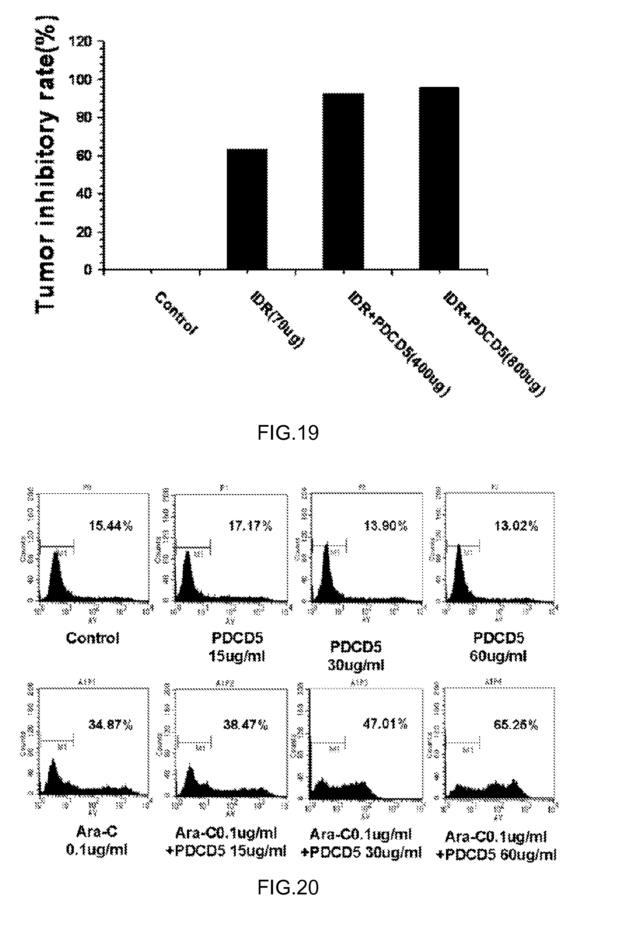

Figure 19 shows the results under the circumstance that the treatments started when the average volume of tumor-bearing mice reached 57.7 mm³, and rhPDCD5 protein was administered intraperitoneally. The results indicate that IDR combined with PDCD5 significantly suppressed tumor growth compared with IDR alone; the tumor inhibitory rate of the treatment with combined rhPDCD5 (800 µg) and IDR was 95.45%, while the tumor inhibitory rate of the treatment of IDR alone was 62.79%. In the treatment with IDR combined with PDCD5, the inhibitory rate of tumor growth increased gradually with the increase of the concentration of PDCD5.
Figure 20 shows the results of FITC/Annexin-V staining and cell apoptosis in vitro measured by flow cytometry, and the results indicate that after the treatment with PDCD5 (60 µg/mL) combined with arabinoside (Ara-c) for 48 hours, K562 cells apoptosis rate was 65.25%, while, in the treatment with Ara-c alone, K562 cells apoptosis rate was only 34.87%.

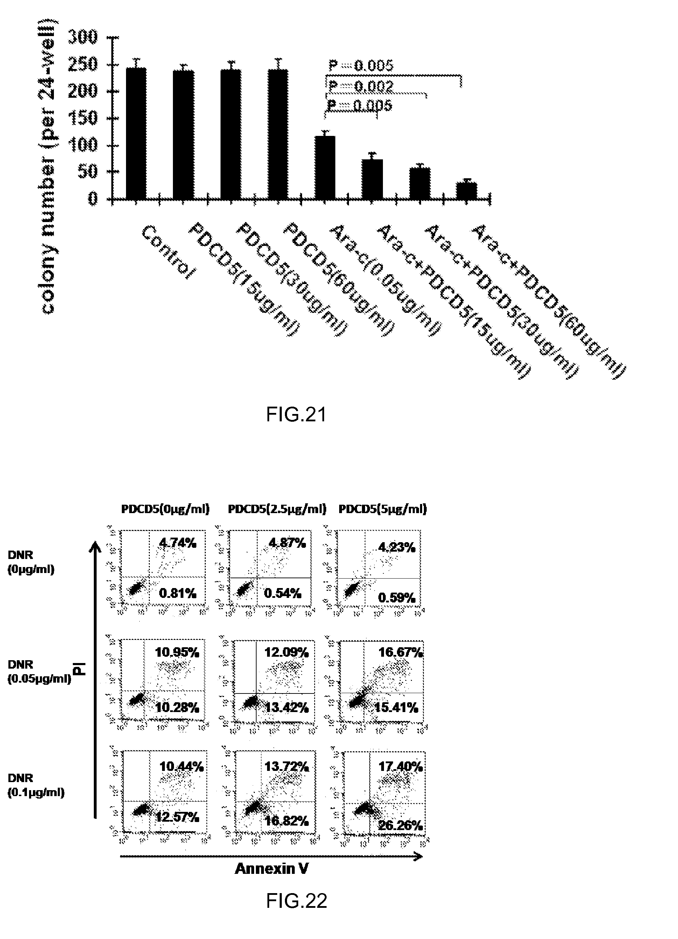

Figure 21 shows the statistics of the numbers of colony formation of K562 cells, and the number of colony formation in the treatment with PDCD5 combined with Ara-c significantly reduced as compared with that in the treatment with Ara-c alone.
Figure 22 shows the results indicating that DNR combined with PDCD5 can enhance DNR induced apoptosis in U937 cells. U937 cells were incubated with DNR in different concentrations or DNR combined PDCD5 for 24h, and the number of apoptotic cells in each group was analyzed by flow cytometry, and percentages of Annexin V positive cells were shown.

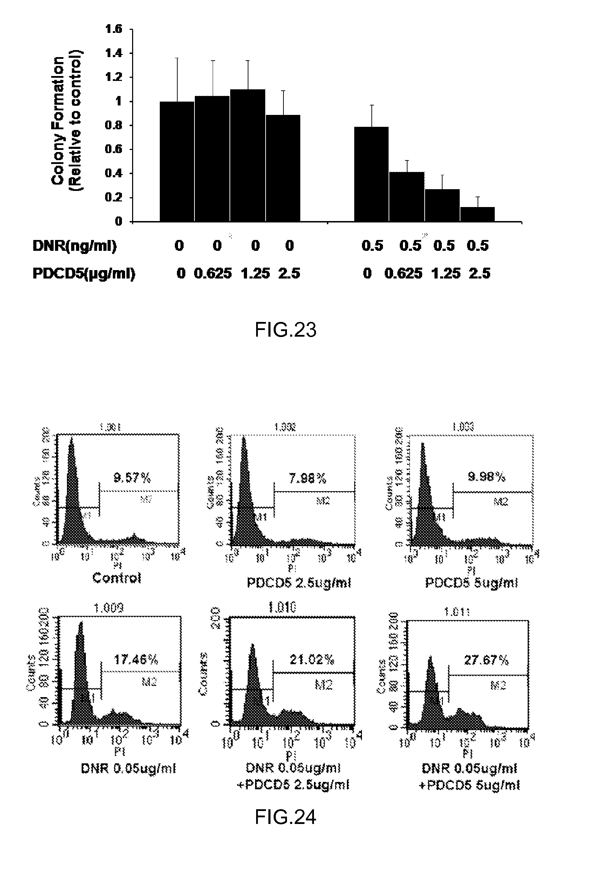

Figure 23 shows the results indicating that the treatment with DNR combined with PDCD5 has stronger colony fomation inhibitory effect in U937 cells comparing to the treatment with DNR alone. U937 cells were treated with indicated concentrations of DNR combined with various concentrations of PDCD5, and colony fomation ability of U937 cells in the semi-solid medium was evaluatied 14 days after the treatments.
Figure 24 shows the results indicating that DNR combined with PDCD5 can enhance DNR induced apoptosis in Raji cells. Raji cells were incubated with DNR in different concentrations or DNR combined PDCD5 for 24h. The number of apoptotic cells in each group was analyzed by flow cytometry, and the percentages of apoptosis cells were shown.

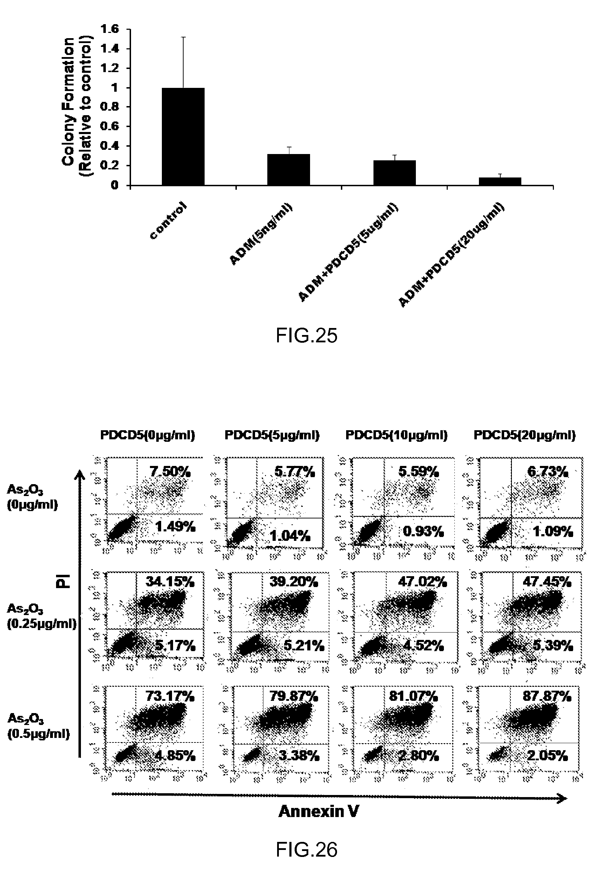

Figure 25 shows the results indicating that adriamycin (ADM) combined with PDCD5 has stronger colony fomation inhibitory effect in Raji cells than ADM alone. Raji cells were treated with ADM (5 ng/mL) combined with different concentrations of PDCD5, and the colony fomation ability of Raji cells in the semi-solid medium was evaluated 14 days after the treatments.
Figure 26 shows the results indicating that arsenious acid (As₂O₃) can enhance As₂O₃ induced apoptosis in HL-60 cells. HL-60 cells were incubated with As₂O₃ in different concentrations or As₂O₃ combined with PDCD5 for 24h. The number of apoptotic cells in each group was analyzed by flow cytometry, while the percentages of Annexin V positive cells were shown.

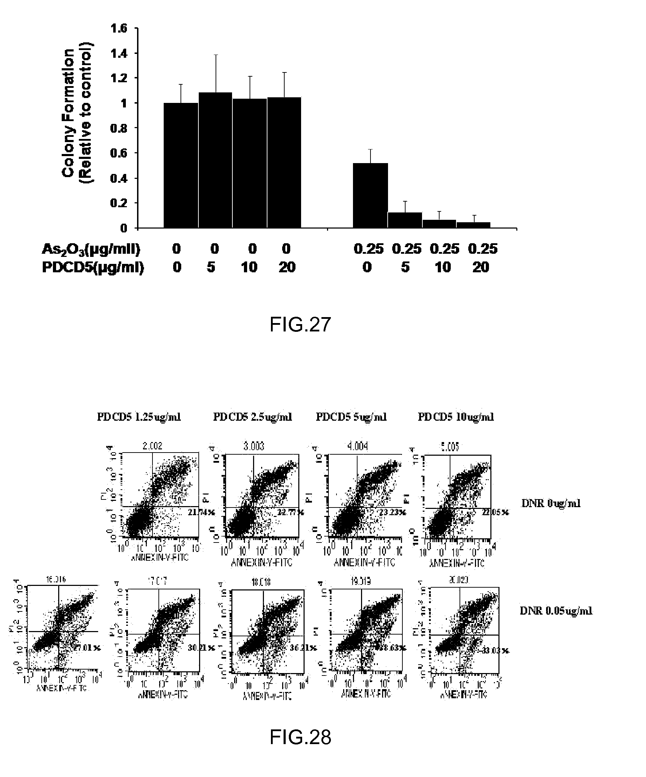

Figure 27 shows the results indicating that arsenious acid (As₂O₃) combined with PDCD5 has stronger colony fomation inhibitory effect in HL-60 cells than As₂O₃ alone. HL-60 cells were treated with the indicated concentrations of As₂O₃ combined with different concentrations of PDCD5, and the colony fomation ability of HL-60 cells in the semi-solid medium was evaluated 14 days after the treatments.
Figure 28 shows the results indicating that rhPDCD5 protein can significantly enhance DNR induced apoptosis in Jurkat cells. In the treatment with DNR and PDCD5 (5 µg/mL), cells apoptosis rate was up to 38.63%, with an increase of 11.62% compared with 27.01% in merely chemotherapeutic treatment.

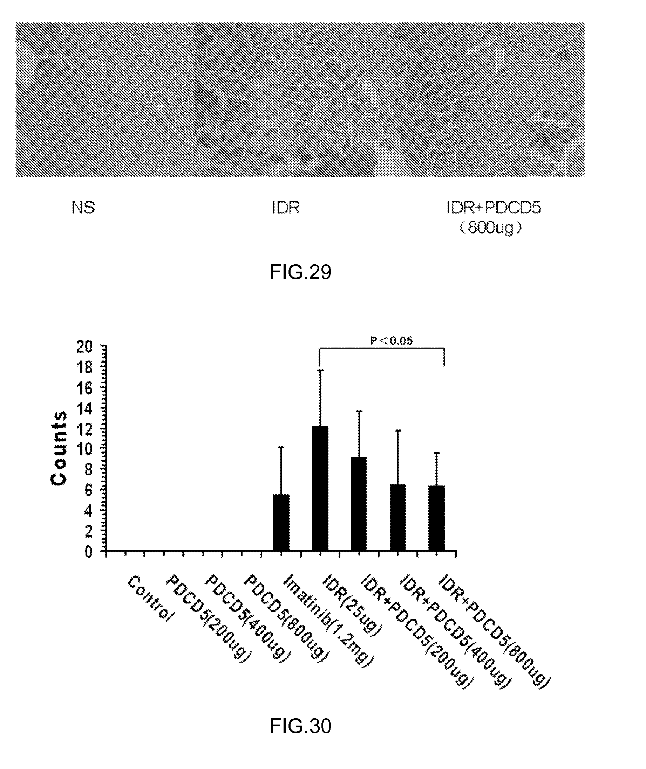

Figure 29 shows representative images of mouse hepatic necrosis after HE staining of mouse liver paraffin sections in each test group. The sinusoidal expansion, congestive hemorrhage and necrosis of liver cells surrounding sinusoid in mouse hepatic tissue was significant after the treatment with chemotherapeutic drug IDR alone, while the sinusoidal expansion, congestive hemorrhage and necrosis of liver cells surrounding sinusoid decreased after the treatment with combined IDR and PDCD5.
Figure 30 shows the statistics when mouse liver paraffin sections were stained by Tunel, as the number of apoptotic cells in five high power microscope fields were counted. The results indicate that the number of apoptotic cells in mice liver in the IDR chemotherapeutic treatment group was significantly higher than that in the treatment group of IDR combined with PDCD5, especially when the concentration of PDCD5 is 800 µg (i.e., 40 mg/kg), the differences of which are statistically significant.

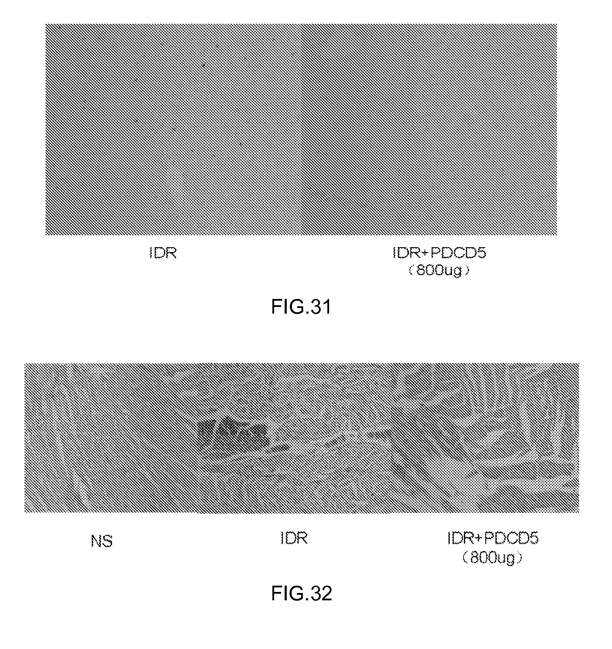

Figure 31 shows representative images of mouse hepatic necrosis after Tunel staining of mouse liver paraffin sections. The number of apoptotic cells in mice liver in the treatment group with chemotherapeutic drug IDR alone was significantly higher than that in the treatment group with IDR combined with PDCD5.
Figure 32 shows representative images of myocardial hemorrhage and congestion , and myofiber fracture after HE staining of mouse heart paraffin sections in each test group. The mouse myocardial hemorrhage and congestion, and myofiber fracture were significant with treatment of chemotherapeutic drug IDR alome, and were reduced in the treatment with combined IDR and PDCD5.

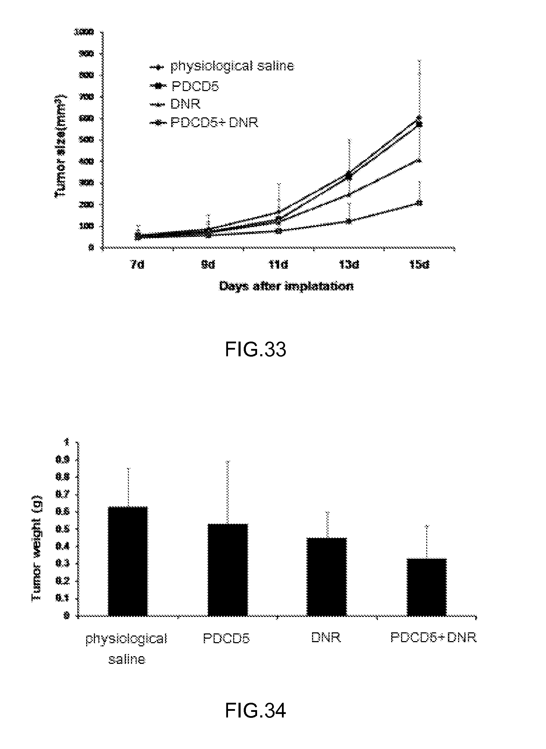

Figure 33 shows tumor growth curves of U937 xenografts in nude mice after different treatments, wherein the results were demonstrated in mean ± standard deviation. The chemotherapeutic drug combined with PDCD5 more significantly inhibited tumor growth compared with chemotherapeutic drug alone.
Figure 34 is a graph showing tumor weight of U937 xenografts in nude mice after different treatments. In the last day of the end of the treatment, tumors were excised and weighed. The weight of tumor in the DNR plus PDCD5 treatment group was decreased compared with the DNR treatment group, and was decreased significantly compared with saline control group (p <0.05).

### DETAILED DESCRIPTION OF THE PREFERRED EMBODIMENTS

The present invention, in one respect, provides the use of PDCD5 polypeptides or polynucleotides that code the PDCD5 polypeptides in preparation of sensitizers of chemotherapeutic agents for cancers, such as liver cancer, cervical cancer, and leukemia. In some embodiments, the PDCD5 polypeptides or the polynucleotides that code the PDCD5 polypeptides deliver the bioactive drugs into cancer cells to have sensitization effect. In other embodiments, the PDCD5 polypeptides or the polynucleotides that code the PDCD5 polypeptides have sensitization effect through promotion of apoptosis. In yet other embodiments, sensitization is achieved via the above two mechanisms. Preferrably, the uses are performed in vivo or in vitro.

The present invention, in another aspect, provides the use of PDCD5 polypeptides or polynucleotides that code the PDCD5 polypeptides in the body of a subject to protect normal or diseased tissues or organs from damages or further damages. In some embodiments, the damage is that caused by trauma and/or occurred inside the body or induced by foreign substances (such as pharmaceutical agents) applied from outside of the body; preferrably, examples of the substances are alcohols, heavy metal salts, toxic metabolites generated from diseases, pharmaceutical agents especially chemotheropeutic agents, toxic substances contained in tobaccos, biological toxicants, chemical toxicants, and any combination thereof. Preferrably, examples of the normal or diseased tissues or organs are lung, spleen, stomach, intestine, Gall bladder cancer, pancreas, liver, heart, and brain, and most preferrably liver and/or heart.

The present invention, in another espect, provides methods of using the PDCD5 polypeptides or the polynucleotide that code the PDCD5 polypeptides to treat cancers, such as liver cancer, cervical cancer, leukemia. The methods include that of applying to subjects the PDCD5 polypeptides or polynucleotides that code the PDCD5 polypeptides in an amount for effective treatment. In some embodiments, the PDCD5 polypeptides or the polynucleotides that code the PDCD5 polypeptides are used in combination with cancer chemotherapeutic agents. In other embodiments, the PDCD5 polypeptides or the polynucleotides that code the PDCD5 polypeptides deliver bioactive drugs into cancer cells to obtain sensitization effect. In other embodiments, the polynucleotides can be in various forms, including but not limited to cloning vector, expression vector, adenovirus, slow virus, plasmid, phasmid, cosmid, and naked DNA, preferrably expression vector such as in the form of nucleic acid vaccine. In yet other embodiments, the PDCD5 polypeptides or the polynucleotides that code the PDCD5 polypeptides provide sensitization effect through promotioin of apoptosis. In yet other embodiments, sensitization is achived via the above two mechanisms. Preferraably, the above discribed methods are applied in vivo or in vitro.

The present invention, in another aspect, provides methods of using PDCD5 polypeptides or polynucleotides that code the PDCD5 polypeptides to protect normal or diseased tissue or organ from damage or further damages, and the methods include that of applying to subjects an effective amount of the PDCD5 polypeptides or polynucleotides that code the PDCD5 polypeptides. In some embodiments, the damage is that caused by trauma and/or induced within the body or by foreign substances (such as pharmaceutical agents) applied from outside of the body; preferrably, examples of the substances are alcohols, heavy metal salts, toxic metabolites generated in diseases, pharmaceutical agents especially chemotheropeutic agents, toxic substances contained in tobaccos, biological toxicants, chemical toxicants, and any combination thereof. Preferrably, examples of the normal or diseased tissues or organs are lung, spleen, stomach, intestine, Gall bladder cancer, pancreas, liver, heart, and brain, and most preferrably liver and/or heart.

The present invention, in another aspect, provides a chemotherapeutic agent sensitizing agent, which contains PDCD5 polypeptides in an amount of effective sensitization.

The present invention, in another aspect, provides a pharmaceutical combination for the treatment of cancer, and the combination contains the PDCD5 polypeptides and chemotherapeutic agents in an amount for effective treatment.

The present invention, in another aspect, provides genetically modified cells used for sensitization of chemotherapeutic agents, and the cells contain PDCD5 polypeptides or polynucleotides that code the PDCD5 polypeptides. In some preferred embodiments, the genetically modified cells are chemotherapy resisting cells, such as cancer cells.

The present invention, in another aspect, provides genetically modified microorganisms used for sensitization of chemotherapeutic agents, while the microorganisms express the PDCD5 polypeptides of sensitization effective amount.

The present invention, in another aspect, provides a pharmaceutical agent used to protect normal or diseased tissue or organ from damage or further damages, while the agent contains an effective amount of the PDCD5 polypeptides.

In some embodiments of the present invention, the PDCD5 polypeptide is selected from the following group: (1) a polypeptide having an amino acid sequence as shown in SEQ No: 1; or (2) a polypeptide having an amino acid sequence with at least 70% of homology to that of the SEQ ID No: 1, the polypeptide having substantially the same biological function or activity comparing to that of the polypeptide of (1); or (3) a functional fragment, variant, analog or derivative of the polypeptide of (1) or (2), having substantially the same biological function or activity comparing to that of the polypeptide of (1) or (2), Wherein, the polynucleotides include: (a) polynucleotides that code the PDCD5 polypeptides of (1), (2) or (3) above; (b) polynucleotides that are hybrided with, under strict conditions, and have at least 70% of consistency comparing to the polynucleotides of (a); and (c) polynucleotide fregments that contain polynucleotides as described in (a) and (b).

Preferrably, in some of the embodiments of the present invention, the PDCD5 polypeptides contain an amino acid sequence, comparing to that of SEQ ID No: 1, with a homology of at least 75%, preferrably at least 80%, more preferrably at least 85%, yet more preferrably at least 90%, even more preferrably at least 95%, and most preferrably at least 97%.

Preferrably, in some of the embodiments of the present invention, the polynucleotides that code the PDCD5 polypeptides have a nucleic acid sequece, comparing to that of SEQ ID No: 2, with a consistancy of at least 70%, preferrably at least 75%, more preferrably at least 80%, at least 85%, at least 90%, at least 95%, and even at least 97%, and most preferrably the polynucleotides have the nucleic acid sequence as shown in SEQ ID No: 2.

In some of the embodiments of the present invention, cancers that can be treated by using tumor chemosensitizers of this invention include: cancers, lymphoma, germ cell tumors, sarcoma, leukemia and lymphoid malignant tumors. The specific examples of the cancers include: squamous cell carcinoma (for example, epithelial squamous cell carcinoma), lung cancer (including small cell lung cancer, non-small cell lung cancer, lung adenocarcinoma, lung squamous carcinoma), peritoneal carcinoma, hepatocellular carcinoma, stomach cancer (including gastrointestinal cancer), pancreas cancer, glioblastoma, cervical cancer, ovarian cancer, liver cancer, bladder cancer, liver cell tumor, breast cancer, colon cancer, rectal cancer, kidney cancer, colorectal cancer, endometrial cancer or uterine cancer, salivary gland cancer, prostate cancer, vulvar cancer, thyroid cancer, anal canal cancer, penile cancer, and head and neck cancer. Preferrably, the cancers are liver cancer, cervical cancer, and leukemia, for example.

In some embodiments of the present invention, the non-restrictive examples of chemotherapeutic agents include cytotoxic agents such as alkylation agents, cyclophosphamides; antimetabolitic agents such as cytarabine, fluorouracil, and arsenic acids; antibiotic agents such as daunorubicin, idarubicin, and doxorubicin; alkaloids such as vinblastine, and camptothecin; steoids such as dexamethasone.

In other embodiments of the present invention, the non-restrictive examples of chemotherapeutic agents include neucleic acid synthesis interferring agemts, such as cytarabine, methotrexate, and fluorouracil; DNA structual and funcion distroying agents such as camptothecin, bleomycins, and cisplatin; DNA intercalating agents that interfere with RNA transcription such as actinomycin D, daunorubicin, and idarubicin; protein synthesis interferring agents such as taxol, vinblastine; hormone balance influencing agents such as tamoxifen; and mitochondria effecting agents, such as arsenic acids.

Preferrably, the chemotherapeutic agents of this invention are selected from antibiotic and antimetabolic agents; preferrably selected from neucleic acid synthesis interferring agents, DNA intercalating agents that interfere with RNA transcription, and mitochondria effecting agents; most preferrably selected from daunorubicin, idarubicin, doxorubicin, cytarabine, and arsenic acids.

In some embodiments of the present invention, the non-restrictive examples of the chemotherapeutic agents include alkylating agents such as thiotepa and cyclophosphamide (CYTOXANTM); alkyl sulfonates such as busulfan, dipropyl amino sulfonate and piposulfan; aziridines such as benzodopa, carboquone, meturedopa, and uredopa; ethylenimines and methylamelamines including hexamethyl melamine, tretamine, trietylenephosphoramide , triethylenethiophosphaoramide, and trimethylolomelamine; mustargens such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas such as carmustine, chlorozotocin, fotemustine, lomustine, nimustine, and ranimustine; antibiotics such as aclacinomysins, actinomycin, authramycin, azaserine, bleomycins, cactinomycin, calicheamicin, carabicin, carnomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, pingyangmycin, potfiromycin, puromycin, quelamycin, rodorubicin, streptonigrin, streptozocin, tubercidin, ubenimex, zinostatin, zorubicin, methotrexate, and 5-fluorouracil (5-FU); folic acid analogues such as denopterin, methotrexate, pteropterin, trimetrexate; purine analogues such as fludarabine, 6-mercaptopurine, thiamiprine, thioguanine; pyridine ananologues such as ancitabine, azacitidine, 6-azauridine, carmofur, cytarabine, dideoxyuridine, doxifluridine, enocitabine, floxuridine, and 5-FU; androgens such as calusterone, dromostanolone propionate, epitiostanol, mepitiostane, and testolactone; anti-adrenergics such as aminoglutethimide, mitotane, and trilostane; folic acid suppliments such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopiraopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside (Ara-C); cyclophosphamide; thiotepa; taxanes, such as paclitaxel (TAXOL®, Bristol-Myers Squibb Oncology, Princeton, NJ.), and docetaxel (TAXOTERE®, Sanofi-aventis); chlorambucil; gemcitabine; 6-thioguanine; mercaptopurine; methotrexate; platium ananologues such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; hormones such as steroid; anthracycline; vinca alkaloids; mithramycin; neocarzinostatin; macromycin; trenimon;a-amanitin; ricin; ricin A-chain; ethiduim bromide; tenoposide; colchicine; dihydroxy anthracin dione; actinomycin D; diphteria toxin; abrin; arbrin A chain; modeccin A chain; α-sarcin; gelonin; mitogellin; retstrictocin; phenomycin; enomycin; aspergillin; ribonuclease; diphtheria toxin; pseudomonas exotoxin; curicin; crotin; calicheamicin; sapaonaria officinalis inhibitor; maytansinoids; goserelin; glucocorticoids; and any pharmaceutically acceptable salts, acids, or derivatives of the above. The chemotherapeudic agents also include antihormon agents that regulateor inhibit the effect of hormon upon tumors; for example, antiestrogen agents including such as tamoxifen, raloxifene, aromatase inhibiting 4(5)-imidazoles, 4-hydroxytamoxifen, trioxifene, keoxifene, LY117018, onapristone, and toremifene (Fareston); and antiandroges such as flutamide, nilutamide, bicalutamide, leuprolide). Preferrably, in some embodiments of this invention, the chemotherapuedic agents are, for example, daunorubicin, doxorubicin, and epirubicin.

The research of this invention showed that recombinant human PDCD5 (rhPDCD5) protein enters into cells via endocytosis mediated by caveolae / lipid rafts. Cells take in the rhPDCD5 protein via temperature dependent endoytosis originated from lipid rafts of cell membrane. The exogenous rhPDCD5 proteins, after endocytosis, may reach the nucleus.

The inventor further observed that the exogenous PDCD5, while entering into cells, shows no specificity of cell types. Among the cells tested so far, for example, HeLa cells, MGC-803 cells, HT-29 cells, A549 cells, PC-3 cells, U937 cells, HL-60 cells, Jurkat T lymphocytes, K562 cells, Raji cells, and TF-1 cells all of them PDCD5 has shown capability of entering into. The rhPDCD5 protein, which can carry macromolecular protein drugs into the cell, is a promising candidate of transport carrier system inside the cells, and can be a tool in research of cell biology and other biotech applications. Especially, the human PDCD5 is a conservative protein in human evolution; PDCD5 expresses in various tissues or organs and can enter into all types of cells, and, comparing to other CPP, has its unique advantages. PDCD5 can promote programmed cell death, and exogenous rhPDCD5 protein can enter into cells; and, consequently, the effect of these two features of PDCD5 is that the PDCD5 protein can enhance the sensitivity of various tumor cells to various chemotherapeutic agents, and thus can be used as tumor chemotherapy sensitizing agents to promote death of tumor cells induced by chemotherapeutic agent and can be used to prepare sensitizing agents of chemotherapeutics.

As used herein, the term "subjects" refers to mammals, human beings for example, but also other animals, such as domestic animals (e.g., dog and cat), livestocks (e.g., cattle, sheep, pig and horse), or experimental animals (e.g., monkey, rat and mouse, rabbit, and guinea pig).

As used herein, the term "polypeptide" refers at least two amino acid residues connected as a chain via covalent bonds such as peptide bonds, and can be recombinant polypeptides, natural polypeptides or synthetic polypeptides.

As used herein, the functional fragments, variants, derivatives and analogues of the PDCD5 polypeptides can be: One or more amino acid residues that are replaced by conservative or non-conservative amino acid residues (preferrably conservative amino acid residues), while the replacing amino acid residues are those encoded or not encoded by genetic code; one or more amino acid residues with substituting groups; a mature polypeptide fused with another compound; one or more other amino acids fused with a mature peptide, such as a sequence or proteinogen sequence to assist purification of a mature protein; insertion and/or addition and/or missing, at one end, or at both ends, or in between, of one or more amino acids. From the contents of the disclosure herein, such fragments, derivatives or ananologues are believed within the scope of the knowledge of an artician skilled in the field.

As used hererin, the terms of "consistency," "percentage consistency," "homology," or "identity" refer to the identity of sequency between two amino acid sequences or nucleic acid sequences. Percentage consistency, which can be determined by comparing two sequences, refers to the number of the same residues (i.e., amino acids or nucleotides) at the same positions shared in the sequences of comparison. The comaparision can be made by using standard algorithms used in the field (for example, Smith and Waterman, 1981, Adv. Appl. Math. 2:482; Needleman and Wunsch, 1970, J. MoI. Biol. 48:443; Pearson and Lipman, 1988, Proc. Natl. Acad. Sci., USA, 85:2444) or a computerized versions of those algorithms (Wisconsin Genetics Software Package Release 7.0, Genetics Computer Group, 575 Science Drive, Madison, WI), while the computerized versions of publicly available are BLAST and FASTA. In addition, ENTREZ available from the National Institutes of Health (Bethesda, MD) can be used for the comparision of the sequences. When using BLAST and Gap-BLAST, the default parameters of each program (BLASTN, for example, available at the website of the National Center for Biotechnology Information) can be used. In an embodiment, the GCG having gap weight of 1 can be used to determine the percentage identity of two sequences, giving weight for the gap of each amino acid as the single amino acid between the two sequences does not match. Alternatively, ALIGN program (version 2.0), as a part of the sequece comparison package GCG (Accelrys, San Diego, CA), can be used.

The term "hybridization" refers to a process of non-covalently combining two single-stranded polynucleotides to form stable double-stranded polynucleotide. The term "hybridization" can also refer to a three-stranded hybridization. Usually, the resultant double-stranded polynucleotide is a "hybrid" or "duplex." "Hybridization conditions" generally include the salt concentration being lower than about 1M, or more preferrably, lower than about 500 mM or lower than about 200 mM. The hybridization temperature can be as low as to 5°C, but usually higher than about 22°C, more frenquently highter than about 30°C, and preferrably higher than about 37°C. The hybridization is usually performed under a strict condition (i.e., the condition under which the probe is hybridized with the target sequence). The strict condition of hybridization is sequence dependent, and will vary with different situations. Longer segments may need higher hybridization temperature for specific hybridization. Because that some other factors, including the composition and length of complementary strand of bases, the existence of organic solvents, and the extent of base mismatch, may influence the degree of hybridization stringency, the combination of parameters is more important than the absolute value of any single parameter. Generally, the strict condition is chosen such that the Tm of specific sequence is about 5°C lower than that under the specified ion concentration and pH. A examplory strict condition includes Na ion (or other salt) concentration of 0.01M to 1M and temperature of at least 25°C when pH is 7.0 to 8.3. For strict conditions, see, for example, Sambrook, Fritsche and Maniatis, "Molecular Cloning, A laboratory Manual," Second Edition, Cold Spring Harbor Press (1989), and Anderson, "Nucleic Acid Hybridization," First Edition, BIOS Scientific Publishers Limited (1999); which, for the above-mentioned purposes, are herein incorporated by reference in their entirty.

### Pharmaceutical composition

The present invention provides also pharmaceutical compositions of the PDCD5 polypeptides (or nucleic acids that code the PDCD5 polypeptides). Besides active ingredients, the pharmaceutical composition of this invention or used based on this invention may contain a pharmaceutically acceptable excipient, carrier, buffer, stabilizer, or other substances publicly known by a person of skill in the art. These substances, should be non-toxic, and should not interfere with the pharmaceutical effect of the active ingredients. The specific characteristics of the carriers or other substances will depend on the routes of administration, which can be, for example, oral, intravenous, or local administration.

The preparations can be of liquid, for example, a physiological salt solution containing a non-phosphorus buffer of pH 6.8∼7.6 or freeze-dried powder.

### Dosage

The PDCD5 polypeptide described herein is administered to an individual preferably in an "effective amount for treatment," "effective amount for sensitization" or "effective amount." The combination is administered to an individual preferably in an "effective amount for treatment." The effective amount for treatment, effective amount for sensitization or effective amount is sufficient to show its benefits for the individual. The actual amount of administration, and the rate and period of administration will depend on the condition of the individual and the severity of the condition. Prescription of the treatment, decision on the doses for example, is made ultimately by a general physician or other physicians based on his professional responsibility in consideration of, usually, the disease to be treated, condition of the individual patient, location of delivery, route of administration, and other factors known to the physician.

In some of the embodiments, the dosage range of the PDCD5 polypeptide can be 30 mg/kg/day to 0.00003 mg/kg/day, or 3 mg/kg/day to 0.0003 mg/kg/day, or 0.3 mg/kg/day to 0.03 mg/kg/day. The dosage ratio of the PDCD5 polypeptide over chemotherapeutic agent can be 1000:1 to 0.001:1, or 100:1 to 0.01:1, or 10:1 1 to 0.1:1.

### Administration

The polypeptides of this invention can be administered alone, but administered preferrably as a pharmaceutical composition, which usually includes an appropriate excipient, diluent or carrier selected based on intented routes of administration. The polypeptide can be used based on the patients to be treated through any appropriate route of administration. Accurate measurement depends on multiple factors, including the specific properties of the polypeptide.

In some of the embodiments of this invention, the PDCD5 polypeptide is administered separately from the chemotherapeutic agent. In other embodiments, the PDCD5 polypeptide is administered together with the chemotherapeutic agent.

Some of the appropriate routes of administration include, but not limited to, oral, rectum, nasal, local (including mouth and sublingual), subcutaneous, vaginal or parenteral (including subcutaneous, intramuscular, intravenous, intradermal , intrathecal and extradural) administration.

For intravenous injection and injection at a disease location, the active ingredient is in the form of a parenterally acceptable aqueous solution having appropriate pH value, isotonia and stability and containing no heat source.

A technical person in the field can well prepare an appropriate solution by using, for example, an isotonic excipient such as sodium chloride injection solution, Ringer's injection solution, or lactic acid Ringer's injection solution. If necessary, preservatives, stabilizers, buffers, antioxidants and/or other additives can be added. The pharmaceutical composition for oral administration can be in the form of tablet, capsule, powder or liquid. The tablet may contain solid carrier, such as gelatin or auxiliary agents. Liquid pharmaceutical composition usually contains liquid carrier, such as water, petroleum, animal or vegetable oil, mineral oil or synthetic oils, and may also contain physiological saline solution, glucose, or other sugar solution or glycols, such as ethylene glycol and propylene glycol or polyethylene glycol.

The examples of technology and scheme as mentioned above or adopted in according to this invention can be found in Remington's Pharmaceutical Sciences, 16th edition, Oslo, A. (ed), 1980.

### Examples

### Example 1: Construction of PDCD5 expression plasmid in E. coli

According to the cDNA and protein sequences (SEQ ID NO:2 and SEQ ID NO:1, respectively) of the PDCD5 coding region, the PDCD5 gene in the plasmid contained in E.coli (deposit number: CGMCC No. 0350) was directionally cloned into the correspoinding NdeI and HindIII recognition sites of pET30a vector (purchased from Novagen) to obtain pET30a-PDCD5 expression vector. After its identity being confirmed, the plasmids was transformed into strain E. coli strain BL21 (Invitrogen Corporation).

### Example 2: Expression and purification of PDCD5 in E. coli.

The pET30a-PDCD5 expression vectors were transformed into E. coli strain BL21 (DE3) and amplified, 1 mM IPTG (isopropyl-β-D-thiogalactoside) (Sigma-Aldrich) was added into the medium to induce rhPDCD5 protein expression. The PDCD5 protein was separated by the method similarly as described in CN1218833A, and purified by ion exchange chromatography with DEAE-Sepharose Fast Flow (Amersham Biosciences) and Hydrophobic Interaction Chromatography (Amersham Pharmacia Biotech). The purified PDCD5 protein was then used in the test as follows.

PAGE electrophoresis was performed for the above-mentioned expression protein samples, as shown in Figure 1. The expression of PDCD5 protein was about 8% as measured by optical density scanning. The protein was mainly expressed in the culture supernatant.

As shown in Figure 2, PAGE electrophoresis was performed for the PDCD5 protein samples in various purifying stages as well as purified PDCD5 protein samples after the above-mentioned two-step purification process of ion-exchange and hydrophobic interaction chromatography. Wherein M is molecular marker, other sample lanes are shown in Figure 2. The arrow indicates the position of recombinant protein PDCD5 band. As shown in the electrophorestogram, the purity of rhPDCD5 protein of over 95% in gel electrophoresis can be obtained.

### Example 3: Kinetics of internalization of PDCD5

HEK293 and U937 cells (ATCC) were maintained in RPMI 1640 (Life Technologies, USA), supplemented with 10% heat-inactivated fetal bovine serum (Hyclone, USA), penicillin (100 units/mL), streptomycin (100 µg/mL), and L-glutamine (2 mM). The HEK293 cells were adherent cells with expression of caveolin-1, while the U937 cells were suspension cells without expression of caveolin-1.

The purified rhPDCD5 protein in the Example 2 was suspended into 0.025 M sodium carbonate-sodium bicarbonate buffer (pH 9.0). The rhPDCD5 protein was labeled (covalent) by fluorescein isothiocyanate (FITC, Sigma-Aldrich) as described by Masseyeff R.F. et al. (Methods Immunology Analysis 3, 237-238, 1993), and then purified by Sephadex G25 to remove free FITC to obtain PDCD5-FITC protein.

Performance of Flow Cytometry-Quantification of internalized FITC-labeled PDCD5 recombinant protein: HEK293 and cells were plated in 24-well plates to about 70% confluence, and then incubated at 37°C with 5 µg/mL recombinant PDCD5-FITC for the times indicated in Figure 3. After incubation, the cells were then washed twice, trypsinized, centrifuged, again washed twice with PBS, and finally analyzed by flow cytometry using a FACSCalibur flow cytometer (BD Biosciences).

As shown in Figure 3, A-D clearly indicates that the PDCD5-FITC protein internalization kinetics in different cell types is similar and effective. As shown in G, however, internalization of PDCD5-FITC, while incubated at 4 °C with both types of cells, was blocked.

Analysis of introcellular localization of the FITC-labeled recombinant PDCD5 protein by fluorescence microscopy : HEK293 cells were grown in specialized glass-bottom microwell dishes (MatTek Corp., USA) to about 50% confluence and washed, and then fresh 10% fetal bovine serum medium and PDCD5-FITC was added until a final concentration of 20 µg/mL. At each time point shown in the drawing, the cells were rinsed twice with PBS buffer and fixed with 2% paraformaldehyde in PBS for 15 min at room temperature. Cells were imaged using a TCS-SP laser-scanning confocal microscope with a 40× or 63 × oil immersion lenses (Leica Microsystems, Mannheim, Germany). Nuclei were visualized by Hoechst 33342 (red regions, a pseudocolor).

As shown in E-F of Figure 3, microscopic analysis of the cells treated with PDCD5-FITC protein (20 µg/mL) indicated that fluorescence was localized to discrete compartments in the cytoplasm (E of Figure 3) with a few labeled proteins reaching the nuclei (F of Figure 3).

### Example 4: PDCD5 molecules can deliver EGFP (green fluorescent protein) into cells

According to the method described by Ying Wang et al. (Journal of Biological Chemistry, vol 281, No. 34, pages 24803-24817, 2006), EGFP-PDCD5 sequence were cloned into the NotI site of pGEX-4T-2 vector to generate pGEX-EGFP-PDCD5, and then were transformed into E. coli strain BL21 (DE3) (Amersham Biosciences), and 50 mM IPTG (isopropy-β-D-thiogalactoside) (Sigma-Aldrich) was added to induce the expression of the corresponding GST-EGFP-PDCD5 fusion proteins. EGFP-rhPDCD5 fusion protein was obtained by purification through glutathione-Sepharose 4B resin (Amer-sham Biosciences).

EGFP protein and PDCD5-EGFP fusion protein were added respectively to the culture supernate of human HEK293 cells for 5 hours and then were analyzed by fluorescence microscope.

As shown in Figure 4, the fluorescence microscopic analysis shows that EGFP-PDCD5 is located in cytoplasm, but EGFP protein cannot. We found that rhPDCD5 is able to lead its EGFP fusion protein into the cells, and from this we know that rhPDCD5 could also lead protein drugs into the cells. All of the above shows the application of rhPDCD5 protein in the preparation of intracellular transport protein drugs.

### Example 5: The rhPDCD5 protein as a chemotherapy sensitizing drug enhances the chemosensitivity of myelogenous leukemia K562 cells to the chemotherapeutic drug idarubicin (IDR) and promotes apoptosis of K562 cells triggered by IDR

Method:
1. Cell staining with FITC/Annexin-V and cell apoptosis in vitro detection by flow cytometry: K562 cells (ATCC) were maintained in RPMI 1640 (Life Technologies , USA) supplemented with 10% FBS (Hyclone, USA), and were plated in 24-well culture dishes to a density of 2×10⁵ cells/mL. rhPDCD5 protein was added with a final concentration of 7.5 µg/mL, 15 µg/mL and 30 µg/mL, respectively, in each well. Chemotherapeutic drug IDR was then added with a final concentration of 56 ng/mL and RPMI 1640 with 10%FBS was added to make the system to 1 mL. After 24 hours of co-culture, the cells were stained with FITC/Annexin-V (Baosai company) and apoptosis was detected by flow cytometry.
2. Clonogenic assay of agarose: Cell concentration was adjusted to 2.5×10⁵ cell/mL. NS (control group) was prepared by taking 0.01 mL single-cell suspension, 0.5 mL 1% agarose, and 0.5 mL 2×RPMI 1640 medium containing 20% FCS, to bring the culture system to 1.0 mL. Test groups were prepared by taking 0.01 mL single-cell suspension and 0.5 mL 1% agarose, and taking IDR and PDCD5 diluted with 2×RPMI 1640 medium containing 20% FCS to an appropriated volume (having a final concentration of 35 ng/mL for IDR, and a final concentration of 15 µg/mL ,30 µg/mL ,or 60 µg/mL for PDCD5 in each respective test group) and further diluted by adding 2×RPMI 1640 medium containing 20% FCS to 0.5 mL, and bringing the culture system to 1.0 mL. The culture system was added on 24-well plates, with 1 mL per well, and three copies of wells for each group. The plates were incubated in a CO₂ incubator for 14 days until colonies formed. The colongenic number was counted by microscope. A colony with more than 30 cells was counted as one positive colony.

Results:
1. As shown in Figure 5, the results of apoptosis detected by flow cytometry indicated that rhPDCD5 protein could significantly promote apoptosis in K562 cells induced by IDR. When the PDCD5 concentration was 30 µg/mL, the cell apoptosis rate of IDR+PDCD5 group was 37.86%, which was 15.29% more than that of IDR group, wherein the cell apoptosis rate was 22.57%.
2. As shown in Figures 6-7, the results of colony formation assay indicated that K562 cell clonogenic number of IDR+PDCD5 obviously decreased comparing to that of IDR alone, and the difference is statistically significant. With the increase of concentration of PDCD5, the clonogenic number of PDCD5+IDR decreased, which is dose-dependent on PDCD5. The data of colongenic inhibition rates indicate that PDCD5+IDR, comparing to IDR alone, can enhance the effect of colongenic inhibition on K562 cell colony formation, and thus PDCD5 significantly increased the inhibition induced by IDR to the formation of the K562 cell colony.

### Example 6: The rhPDCD5 protein, as a chemotherapy sensitizing drug, can significantly enhance the effect of idarubicin on myelogenous leukemia K562 in vivo

Method: BALB/c nude female mice (6-8 weeks of age) were used, and the mice were pretreated by i.p. injections of cyclophosphamide (Jiangsu Hengrui Medicine Co., Ltd.) once daily at a dose of 2 mg per mouse for two consecutive days. After two days of the cyclophosphamide injection, 1.5×10⁷ K562 cells were injected subcutaneously into the single flanks of the mouse. The mice bearing K562 cell xenografts were divided randomly into five groups: Normal saline, irrelevant protein, rhPDCD5, IDR (idarubicin, Sigma Company), and IDR plus rhPDCD5. IDR treatments were given every other day by i.p. The longest diameter and shortest diameter of tumors were measured by vernier caliper at the beginning of treatment and during the treatment. The side effects were observed during the treatment. At the end of the experiment, the mice were killed and tumor massed were dissected and then weighted. Tumor inhibition rate was calculated as: (Tumor weight of control group - tumor weight of test group) / tumor weight of control group × 100%. Tumor volume was calculated using the following formula: πls²/6, where l= long diameter and s = short diameter.

Results: In vivo test showed that the animals' tumor massed appeared 7 to 9 days after inoculated subcutaneously with K562 cells. The tumor volume of PDCD5+IDR group was smaller than that of the control group and other test groups; the growth of tumor of PDCD5+IDR group was slower. When the dose of IDR was constant, the inhibition of tumor growth was dose-dependent on PDCD5 protein. In the treatment of PDCD5 protein intratumoral injections, tumor growth of the test group with 70 µg IDR and 60 µg PDCD5 protein was slower than that of the test group with 70 µg IDR alone, meanwhile, tumor volume and weight was significantly reduced significantly. The tumor inhibition rate of the test group with 70 µg IDR and 60 µg PDCD5 protein was 92.02%. In each test group, n (number of mice ) = 6. In the treatment with systemic administration of PDCD5 protein by intraperitoneal injection, the treatment started when the average tumor size was 140 mm³. The tumor inhibition rate of 25 µg IDR group was 22.67%, and that of 25 µg IDR + 800 µg PDCD5 protein group was 75.3%, while in the same batch of experiments with 6 mice for each group, the tumor inhibition rate of treatment with daily oral administration of 1.2 mg Imatinib was 40.82%.. In aother batch of experiments with 4 mice for each group, the treatment started when the average tumor size was 57.7mm³, and the tumor inhibition rate of 70 µg IDR group was 62.79% while that of 70 µg IDR + 800 µg PDCD5 protein group was 95.45%. (See Figures 8-19).

### Example 7: The rhPDCD5 protein as a chemotherapy sensitizing drug enhances the chemosensitivity of myelogenous leukemia K562 cells to the chemotherapeutic drug arabinoside (Ara-C)

Method:
1. Cell staining with FITC / Annexin-V and cell apoptosis detection in vitro by flow cytometry: K562 cells (ATCC) were maintained in RPMI 1640 (Life Technologies, USA) supplemented with 10% FBS (Hyclone, USA), and were plated in 24-well culture dishes to a density of 2×10⁵ cells per well. rhPDCD5 protein was added in each well with a final concentration of 15 µg/mL, 30 µg/mL and 60 µg/mL, respectively. Chemotherapeutic drug Ara-C (Pharmacia, Italia) was then added with a final concentration of 0.1 µg/mL and RPMI 1640 medium supplemented with 10% FBS was added to make the culture system to 1 mL. After incubation for 48 hours, the cells were stained with Annexin-V/PI (Biosea) and then were analyzed for apoptosis by flow cytometry.
2. Clonogenic assay of agarose: The concentration of K562 cells was adjusted to 2.5×10⁵ cell/mL. NS (control group) was prepared by taking 0.01 mL single-cell suspension, 0.5 mL 1% agarose, and 0.5 mL 2×RPMI 1640 medium with 20% FCS, to bring the culture system to 1.0 mL. Test groups were prepared by taking 0.01 mL single-cell suspension and 0.5 mL 1% agarose, and taking Ara-c and PDCD5 diluted with 2×RPMI 1640 medium containing 20% FCS to an appropriate volume (having a final concentration of 35 ng/mL for Ara-c, and a final concentration of 15 µg/mL, 30 µg/mL, or 60 µg/mL for PDCD5 in each respective test group) and further diluted by adding 2×RPMI 1640 medium containing 20% FCS to 0.5 mL, and bringing the culture system to 1.0 mL. The samples were plated on 24-well plates, with 1 mL per well, and 3 copies of wells for each group, and the plates were incubated in a CO₂ incubator for 14 days until colonies formed. The clonegenic number was counted by microscope. A colony with more than 30 cells was counted as one positive colony, while the rate of colongenic inhibition of 3-well average was calculated as: Rate of colongenic inhibition = (1 - colongenic number of test group / colongenic number of control group).

Results:
1. As shown in Figure 20, rhPDCD5 protein could significantly promote apoptosis in K562 cells induced by Ara-C. When the concentration of PDCD5 is 60 µg/mL, the apoptosis rate of Ara-C+PDCD5 group was 65.25%, with 30.38% increase in apoptosis rate compared with 34.87% of Ara-C alone group.
2. As shown in Figure 21, K562 cell clonogenic inhibition of Ara-c + PDCD5 group is significantly higher than that of Ara-c alone group. With the increase of concentration of PDCD5, the clonogenic inhibition rate of PDCD5+Ara-c group increases and is dose-dependent on PDCD5.

### Example 8: The rhPDCD5 protein as a chemotherapy sensitizing drug enhances the chemosensitivity of histiocytic lymphoma U937 cells to the chemotherapeutic drug daunorubicin (DNR) and promotes apoptosis of U937 cells triggered by DNR

Cell apoptosis assay: U937 cells (ATCC), in the logarithmic phase, were maintained in RPMI 1640 (Life Technologies, USA) supplemented with 10% FBS (Hyclone, USA), and were adjusted to a density of 2×10⁵ cells/mL and then were divided into control groups and test groups. U937 cells in the control groups were treated with DNR (Pharmacia Italia SPA) alone, with the final concentrations of 0 µg/mL, 0.05 µg/mL, and 0.1 µg/mL. U937 cells in the test groups were treated with DNR of various concentrations combined with PDCD5 of various concentrations, with the final concentrations of PDCD5 being 0 µg/mL, 2.5 µg/mL, and 5 µg/mL, respectively. After incubated for 24 hours in a 37°C, 5% CO₂ and saturatedly humidified incubator, the cells were stained with Annexin-V/PI (Biosea) and then were analyzed for apoptosis with a FACSCalibur flow cytometer.

Analysis for colony forming ability (CFA): U937 cells (ATCC), in the logarithmic phase, were maintained in RPMI 1640 medium supplemented with 20% FBS and 0.4% methyl cellulose (Sigma) by the semi-solid colony culture method and were adjusted to a density of 5×10³ cells/mL. The cells were divided into control groups and test groups, and each group was assayed in triple wells. U937 cells in the control groups were treated with DNR alone, with the final concentrations of 0 ng/mL and 0.5 ng/mL. U937 cells in the test group were treated with DNR of various concentrations combined with PDCD5 of various concentrations, while the final concentrations of PDCD5 were 0 µg/mL, 0.625 µg/mL, 1.25 µg/mL, and 2.5 µg/mL, respectively. After incubation for 14 days in a 37°C, 5% CO₂ and saturatedly humidified incubator, a colony with more than 30 cells was counted by an inverted microscope in order to analyze colony forming ability of each group. The colony forming ability was shown as the ratio of average number of colony in each group versus that of the untreated control group in which the final concentrations of DNR and PDCD5 were 0 µg/mL.

As shown in Figure 22, rhPDCD5 can significantly enhance DNR-induced apoptosis in U937 cells. In addition, PDCD5 at the final concentration of 5 µg/mL can more markedly increase the susceptibility of tumor cells to cytotoxic drugs-induced apoptosis than that at the final concentration of 2.5 µg/mL. When the final concentration of PDCD5 was 5 µg/mL, the rate of apoptosis in the test group of low-dose DNR (0.05 µg/mL) plus PDCD5 was 32.08%, with an increase of 10.85% compared with 21.23% of DNR group; the rate of apoptosis in the test group of high-dose DNR (0.1 µg/mL) plus PDCD5 was 43.66%, with an increase of 20.65% compared with 23.01% of DNR group.

As shown in Figure 23, rhPDCD5 can significantly enhance the inhibition of CFA by DNR in U937 cells. In U937 cells, the CFA of cells treated with 0.5 ng/mL DNR alone was 79%, but the CFA of cells treated with DNR plus rhPDCD5 decreased as the rhPDCD5 concentration increased and the CFAs were 41 %, 27%, 12%, respectively. The comparision of CFAs of DNR plus rhPDCD5 co-treatment groups with that of the DNR alone group is statistically significant, with P<0.05. rhPDCD5 alone did not have significant effect on the CFA of U937 cells.

### Example 9: As a chemosensitizer for tumor chemotherapy, the rhPDCD5 can sensitize Raji cells to DNR-induced apoptosis, and promote the apoptosis of Raji

Raji cells were cultured in RPMI 1640 medium (Life Technologies, USA) supplemented with 10% Fetal Bovine Serum (FBS, Hyclone, USA). The cells were seeded in 24-well plates with 2×10⁵ cells per well. rhPDCD5 was added to each well and the final concentration were 0 µg/mL, 2.5 µg/mL and 5 µg/mL, respectively. Subsequently, DNR was added and the final concentration were 0 µg/mL and 0.05 µg/mL, and RPMI 1640 medium supplemented with 10% FBS was added to ensure the system was brought to 1 mL. After 24h incubation, the cells were stained with FITC/Annexin-V (Beijing Biosea Biotechnology Co., Ltd.) and the appoptosis was detected by flow cytometry; as shown in Figure 24, rhPDCD5 can significantly enhance DNR-induced apoptosis in Raji cells. When the concentration of PDCD5 was 5 µg/mL, the rate of apoptosis was 27.67%, with an increase of 10.21% compared with 17.46% of DNR group.

### Example 10: As a chemosensitizer for tumor chemotherapy, the rhPDCD5 can sensitize Raji cells to ADM-induced cytotoxicity

Colony-forming ability (CFA) assay: Raji cells of logarithmic growth phase were cultured, and the method was the same as that in Example 8. Divided into four groups (each with 3 wells): control group, ADM (Pfizer Pharmaceuticals Limited, at the final concentration of 5 ng/mL) group, ADM plus low-dose PDCD5 (5 µg/mL) group and ADM plus high-dose PDCD5 (20 µg/mL) group. Cultures were incubated in a 37°C, 5% CO₂ and saturatedly humidified incubator for 14 days. The numbers of colonies (containing ≥ 30 cells) were counted by inverted microscope to observe the CFA, which is indicated by the ratio of everage number of colony formation in each of the test groups versus that of the untreated control group.

As shown in Figure 25, rhPDCD5 protein can significantly enhance the inhibition of CFA by ADM in Raji cells. CFA of the cells treated with ADM alone was 32%, but the CFAs of the cells treated with ADM plus rhPDCD5 decreased as the rhPDCD5 concentrations increased and the CFAs were 2.5% and 7.8%, respectively. The CFAs of ADM plus high-dose rhPDCD5 co-treatment group compared with that of the ADM alone group are statistically significant with P<0.05.

### Example 11: As a chemosensitizer for tumor chemotherapy, the rhPDCD5 can sensitize HL60 cells to arsenious acid (As₂O₃) induced cytotoxicity, and promote the apoptosis of HL60

Cell apoptosis assay: HL-60 cells (ATCC) of logarithmic growth phase were cultured and the density of cells was 2 × 10⁵/mL. The cells were divided into control and test groups. In the control groups, the HL60 cells were treated only with As₂O₃ (Harbin Yida Pharmaceutical Co., Ltd.), and the final concentration of As₂O₃ were 0 µg/mL, 0.25 µg/mL and 0.5 µg/mL, respectively. In the test groups, the HL60 cells were treated with As₂O₃ in the various concentrations combined with PDCD5 in various concentrations, the final concentrations of PDCD5 being 0 µg/mL, 5 µg/mL, 10 µg/mL and 20 µg/mL, respectively. The cells were incubated in a 37°C, 5% CO₂ and saturatedly humidified incubator for 24h, and subsequently were stained with Annexin-V/PI and detected for apoptosis by flow cytometer.

Colony-forming ability (CFA) assay: HL60 cells of logarithmic growth phase were cultured by a method identical to that in Example 8. The cells were divided into control and test groups (each with 3 wells): In the control groups, the HL60 cells were treated only with arsenic trioxide and the final concentration of which were 0 µg/mL and 0.25 µg/mL, respectively; in the test groups, the HL60 cells were treated with different concentrations of As₂O₃, which was combined with different concentrations of PDCD5, the final concentrations of PDCD5 were 0 µg/mL, 5 µg/mL, 10 µg/mL and 20 µg/mL, respectively. Cultures were incubated in a 37°C, 5% CO₂ and saturatedly humidified incubator for 14 days. The numbers of colonies (containing ≥ 30 cells) were counted on day 14 by inverted microscope. The colony-forming ability was observed as the ratio of the number of average colony formation in the test groups versus that in the untreated control group. In the untreated control group, the final concentration of both As₂O₃ and PDCD5 was 0 µg/mL.

As shown in Figure 26, the rhPDCD5 can significantly enhance As₂O₃-induced apoptosis in HL60 cells. In addition, the apoptosis rates of cells were enhanced gradually with the increase of concentration of rhPDCD5. When the final concentration of PDCD5 was 20 µg/mL, the rate of apoptosis in the test group of low-dose As₂O₃ (0.25 µg/mL) plus PDCD5 was 52.84%, with an increase of 13.52% compared with 21.23% of As₂O₃ group; the rate of apoptosis in the test group of high-dose As₂O₃ (0.5 µg/mL) plus PDCD5 was 43.66%, with an increase of 11.9% compared with 78.02% of As₂O₃ group.

As shown in Figure 27, the rhPDCD5 can significantly enhance the CFA inhibition by As₂O₃ in HL60 cells. When treated with As₂O₃ (0.25 µg/mL) alone, the CFA of the cells was 52%, but when PDCD5 was combined in the treatment, the colony formation significantly reduced. In addition, the apoptosis rates of cells were enhanced with the increase of concentration of rhPDCD5, and the CFAs of cells were 13 %, 7% and 5%, respectively. The comparision between the CFAs of As₂O₃ plus high-dose rhPDCD5 co-treatment group and that of the As₂O₃ alone group are statistically significant, with P<0.05. The rhPDCD5 alone had no significant affect on the CFA of HL60.

### Example 12: As a chemosensitizer for tumor chemotherapy, the rhPDCD5 can sensitize Jurkat cells to DNR-induced cytotoxicity, and promote the apoptosis of Jurkat

The density of Jurkat cells were adjusted by RPMI 1640 medium (Life Technologies, USA) supplemented with 10% Fetal Bovine Serum (FBS, Hyclone, USA). The cells were seeded in 24-well plates with 2×10⁵ cells per well. rhPDCD5 protein was added to each well with the final concentration being 1.25 µg/mL, 2.5 µg/mL and 10 µg/mL, respectively.

Subsequently, DNR ( Pharmacia Italia S.P.A ) was added with its final concentration of 0.05 µg/mL, and RPMI 1640 medium supplemented with 10% FBS was added to ensure the system to be 1 mL. After 24h incubation, the sample was stained with FITC/Annexin-V (Beijing Biosea Biotechnology CO., LTD) and detected for apoptosis by flow cytometry.

As shown in Figure 28, rhPDCD5 can significantly enhance DNR-induced apoptosis in Jurkat cells. When the concentration of PDCD5 was 5 µg/mL, the rate of apoptosis in group treated with DNR plus PDCD5 was 38.63%, with an increase of 11.62% compared with 27.01 % of DNR group.

### Example 13: In vivo, the rhPDCD5 protein had protective function on animals' livers in chemotherapy

Method: In BALB/c nu/nu female mice (6-8 weeks of age) was injected intraperitoneally cyclophosphamide (CTX) (Jiangsu Hengrui Medicine Co., Ltd.), 2 mg/day per mouse for two days. One day after the second cyclophosphamide injection, 1.5×10⁷ of K562 cells were injected subcutaneously into the single armpit of each of the mice. The mice bearing K562 cell xenografts were divided randomly into nine groups (n = 6, i.e., six mice in each group) and each of the mice was i.p. treated, respectively, with normal saline, PDCD5, IDR (Sigma Corporation), and IDR plus PDCD5. Normal saline, PDCD5 (200 µg, 400 µg, or 800 µg, i.e., 10 mg/kg, 20 mg/kg, or 40 mg/kg, respectively), IDR (25 µg, i.e., 1.25 mg/kg) and IDR (25 µg, i.e., 1.25 mg/kg) plus PDCD5 (200 µg, 400 µg, or 800 µg, i.e., 10 mg/kg, 20 mg/kg, or 40 mg/kg, respectively) were used for intraperitoneal injection every other day for a total of five treatments. Imatinib was given orally, at a dosage of 1.2 mg (i.e., 60 mg/kg) for a total of 10 days. At the end of the experiment, mice were killed. The mice were the same batch of that of Example 6.

The excised liver of mice after treatment were fixed in 10% neutrally buffered formalin and then embedded in paraffin after dehydration. The paraffin embedding were cut and paraffin sections after HE staining were used to observe the necrosis of experimental mouse livers. Meanwhile, paraffin sections after Tunel (Roche Inc.) staining were used to observe the apoptosis of experimental mouse livers, and then the number of apoptotic cells was counted in five high-power microscope field for statistical analysis.

Results: The paraffin sections after HE staining (Table 1, Figure 29) showed that the sinusoidal expansion, congestive hemorrhage and necrosis of liver cells surrounding sinusoid of livers in mice only treated with chemotherapeutic drugs were obvious. However, the sinusoidal expansion, congestive hemorrhage and necrosis of liver cells surrounding sinusoid were significantly reduced when rhPDCD5 protein was combined with chemotherapeutic drugs of the treatment. As shown in Figure 30-31, in Tunel assay, the number of apoptotic cells was counted under five high- power fields for statistical analysis, and it is found that the number of apoptotic liver cells was significantly higher in the treatment with chemotherapeutic drugs alone comparing with the treatment of rhPDCD5 protein combined with chemotherapeutic drugs, especially when the therapeutic dose of PDCD5 protein was 800 µg (i.e., 40 mg/kg); the difference here are statistically significant. The results indicate that when rhPDCD5 protein was combined with chemotherapeutic drugs for treatment, the rhPDCD5 protein, as a chemosensitizer for tumor chemotherapy, can sensitize K562 cells to IDR-induced treatment, meanwhile, the rhPDCD5 protein can reduce the chemotherapy side effects on liver and protect the liver from chemotherapy toxicity, and thus is protective for the livers of animals.

**Table 1. The paraffin sections of mouse livers in each group after HE staining were used to observe the sinusoidal expansion, congestive hemorrhage and necrosis of liver cells surrounding sinusoid of livers (figures therein indicate the number of mice).**

| | Sinusoidal expansion, congestive hemorrhage and necrosis of liver cells surrounding sinusoid | | | |
|---|---|---|---|---|
| | ++ | + | +/- | - |
| Control | 0 | 0 | 0 | 6 |
| PDCD5(200 µg) | 0 | 0 | 0 | 6 |
| PDCD5(400 µg) | 0 | 0 | 0 | 6 |
| PDCD5(800 µg) | 0 | 0 | 0 | 6 |
| Imatinib(1.2 mg) | 0 | 2 | 0 | 4 |
| IDR(25 µs) | 3 | 3 | 0 | 0 |
| IDR(25 µg)+ PDCD5(200 µg) | 0 | 4 | 0 | 2 |
| IDR(25 µg)+ PDCD5(400 µg) | 0 | 3 | 0 | 3 |
| IDR(25 µg)+ PDCD5(800 µg) | 0 | 3 | 1 | 2 |

### Example 14: The rhPDCD5 protein shown in vivo protective effect to heart in tumor chemotherapy

Method: In BALB/c nu/nu female mice (6-8 weeks of age) was injected intraperitoneally cyclophosphamide (CTX) (Jiangsu Hengrui Medicine Co., Ltd.), 2 mg/day per mouse for two days. One day after the second cyclophosphamide injection, 1.5×10⁷ of K562 cells were injected subcutaneously into the single armpit of each of the mice. The mice bearing K562 cell xenografts were divided randomly into nine groups (n = 6, i.e., six mice in each group) and each of the mice was i.p. treated, respectively, with normal saline, PDCD5, IDR (Sigma Corporation), and IDR plus PDCD5. Normal saline, PDCD5 (200 µg, 400 µg, or 800 µg, i.e., 10 mg/kg, 20 mg/kg, or 40 mg/kg, respectively), IDR (25 µg, i.e., 1.25 mg/kg) and IDR (25 µg, i.e., 1.25 mg/kg) plus PDCD5 (200 µg, 400 µg, or 800 µg, i.e., 10 mg/kg, 20 mg/kg, or 40 mg/kg, respectively) were used for intraperitoneal injection every other day for a total of five treatments. Imatinib was given orally, at a dosage of 1.2 mg (i.e., 60 mg/kg) for a total of 10 days. At the end of the experiment, mice were killed. The mice were the same batch of that of Example 6.

The excised heart of mice after treatment were fixed in 10% neutrally buffered formalin and then embedded in paraffin after dehydration. The paraffin embedding were cut and paraffin sections after HE staining were used to observe the myocardial congestion, hemorrhage, and myofiber degeneration of heart in mice.

Results: The paraffin sections after HE staining (Table 2, Figure 32) showed that the myocardial congestion, hemorrhage, and myofiber degeneration of hearts in mice of the groups treated with chemotherapeutic drugs alone were obvious. However, the myocardial congestion, hemorrhage, and myofiber degeneration of hearts was significantly reduced when rhPDCD5 protein was combined with chemotherapeutic drugs in the treatment. This shows that when rhPDCD5 protein was combined with chemotherapeutic drugs in the treatment, the rhPDCD5 protein, as a chemosensitizer for tumor chemotherapy, can sensitize K562 cells to IDR-induced treatment, meanwhile, the rhPDCD5 protein can reduce the chemotherapy side effects on the heart, protect the hearts from chemotherapy toxicity, and thus have obvious protective function on animals' hearts.

**Table 2 The paraffin sections of mice hearts in each group after HE staining were used to observe the myocardial congestion, hemorrhage, and myofiber degeneration of hearts (figures indicate the number of mice).**

| | Myocardial hemorrhage and congestion | | | Myofiber degeneration | |
|---|---|---|---|---|---|
| | ++ | + | - | + | - |
| Control | 2 | 1 | 3 | 0 | 6 |
| PDCD5(200 µg) | 0 | 0 | 6 | 0 | 6 |
| PDCD5(400 µg) | 0 | 0 | 6 | 0 | 6 |
| PDCD5(800 µg) | 0 | 0 | 6 | 0 | 6 |
| Imatinib(1.2 mg) | 1 | 1 | 4 | 0 | 6 |
| IDR(25 µg) | 3 | 3 | 0 | 3 | 3 |
| IDR(25 µg)+ PDCD5(200 µg) | 3 | 2 | 1 | 2 | 4 |
| IDR(25 µg)+ PDCD5(400 µg) | 2 | 3 | 1 | 2 | 4 |
| IDR(25 µg)+ PDCD5(800 µg) | 1 | 0 | 5 | 0 | 6 |

### Example 15: The rhPDCD5 as a cancer chemotherapy sensitizing drugs can significantly enhance the therapeutic effect of DNR on the U937 leukemia in vivo

U937 nude mice xenograft model: BALB/c nude mice (5∼6 weeks of age) were pretreated with cyclophosphamide (100 mg/kg, injected intraperitoneally once a day for two consecutive days). On day 3, 1×10⁷ (100µl) of U937 cells were subcutaneously (s.c.) injected into a single flank of each of the mice. The treatments began on day 7 after inoculation when the average tumor diameter reached about 5 mm. The mice were divided randomly into four groups (n = 6, i.e., 6 mouses in each group) and treated with normal saline (NS), rhPDCD5 (40 µg, locally injected into the tumor), DNR (40 µg, intraperitoneal) or DNR (40 µg) plus rhPDCD5 (40 µg), once a day for 8 consecutive days.

Tumor volume (V) was determined every other day by vernier caliper measurements and calculated based on the equation of V = ab²/2, where "a" refers to a long tumor diameter and "b" refers to a short diameter, then tumor volume growth curve was drawn. The percentage of reductions in tumor growth was calculated using the formula: [1-(Tf-Ti)/(Cf-Ci)]×100, where Tf and Ti are the final and initial mean tumor volumes, respectively, of the group receiving a specific treatment, and Cf and Ci are the final and initial mean tumor volumes, respectively, of the NS control group. After the treatment, tumors were collected and weighed and tissues were sectioned and processed by histological hematoxylin and eosin (H&E) staining. Results were statistically analyzed using SPSS 13.0. The measurements were expressed as the mean ± standard deviation. Data were analyzed by the single-factor ANOVA and Student's *t* est and are presented as mean ± standard deviation (SD). P<0.05 was considered statistically significant.

At the time point from the eleventh day to the fifteenth day, there were obvious differences in tumor size between the DNR plus rhPDCD5 co-treatment group and the DNR treatment group, especially on the fifteenth day (Fig. 33). Table 3 showed the average tumor volumes in different groups on the fifteenth day. We found that the DNR plus rhPDCD5 combinational chemotherapy could significantly reduce tumor growth in the U937 xenografts. After treatment on day 15, we assessed the anti-tumor effects by the percentage of inhibition on tumor sizes in comparison to the control group. The inhibitions of tumor growth in the DNR group and DNR plus rhPDCD5 group were 31.97% and 65.46%, respectively. The average tumor weight of saline group was 0.63±0.22g. DNR group and DNR + PDCD5 treatment group showed inhibition of tumor growth, and the average tumor weight reduced by 0.45 ± 0.15g, 0.36 ± 0.22g, respectively. These results suggest that PDCD5 combined DNR treatment compared with DNR alone could significantly inhibit tumor growth (p <0.05).

**Table 3 The average tumor size of different groups (mm³)**

| Group | Average size of tumors |
|---|---|
| | (mean ± SD) |
| 1 (saline) | 603.06 ± 266.37 |
| 2 (PDCD5) | 571.96 ± 237.91 |
| 3 (DNR) | 410.27 ± 154.93* |
| 4 (PDCD5 + DNR) | 208.37 ± 100.46**#▲ |

| | |
|---|---|
| Notes: * represents P < 0.05 when comparing with group 1; ** represents P< 0.01 when comparing with group 1; # represents P < 0.05 when comparing with group 3; ▲ represents P< 0.01 when comparing with group 2. | |

## Claims

1. Use of a PDCD5 polypeptide or a polynucleotide that codes the PDCD5 polypeptide in preparation of a sensitizer of a chemotherapeutic agent for cancer, including liver cancer, cervical cancer, and leukemia, of a subject, wherein the PDCD5 polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).

2. The use according to claim 1, wherein the polynucleotide compries:
(a) a nucleotide sequence coding an amino acid sequence as shown in SEQ ID No: 1;
(b) a nucleotide sequence hybridsed with a nucleotide sequence described in (a) under strict conditions; or
(c) a nucleotide sequence having at least 70%, 75%, 80%, 85%, 90%, 95% or 97% of consistency comparing to the nucleotide sequence as described in (a).

3. The use according to claim 1, wherein the chemotherapeutic agent is selected from antibiotic agents, antimetabolic agents, or any combination thereof; preferably selected from neuceic acid synthesis interferring agents, DNA intercalating agents that interfere with RNA transcription, and mitochondria effecting agents or any combination thereof; most preferrably selected from daunorubicin, idarubicin, doxorubicin, cytarabine, and arsenic acids or any combination thereof.

4. Use of a PDCD5 polypeptide or a polynucleotide that codes the PDCD5 polypeptide in preparation of pharmaceutical agents to protect normal or diseased tissue or organ of a subject from damage or further damages, preferably the damage being that caused by trauma and/or occurred inside of body or induced by foreign substances, such as a pharmaceutical agent, applied from outside of body, more preferably the foreign substances being selected from alcohols, heavy metal salts, toxic metabolites generated from diseases, pharmaceutical agents especially chemotheropeutic agents, toxic substances contained in tobaccos, biological toxicants, chemical toxicants, and any combination thereof, wherein the polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).

5. The use according to claim 4, wherein the polynucleotide compries:
(a) a nucleotide sequence coding an amino acid sequence as shown in SEQ ID No: 1;
(b) a nucleotide sequence hybridsed with the nucleotide sequence described in (a) under strict conditions; or
(c) a nucleotide sequence having at least 70%, 75%, 80%, 85%, 90%, 95% or 97% of consistency comparing to the nucleotide sequence as described in (a).

6. The use according to claim 4, wherein normal or diseased tissue or organ is heart or liver

7. A pharmaceutical combination comprising a chemotherapeutic agent selected from antibiotic agents, anti-metabolic agents, or any combination thereof, and a PDCD5 polypeptide or polynucleotide that codes the PDCD5 polypeptide in an amount of effective sensitization, wherein the PDCD5 polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).

8. The pharmaceutical combination according to claim 7, wherein the PDCD5 polypeptide is coded by a polynucleotide of a nucleotide sequence selected from the group consisting of:
(a) a nucleotide sequence coding an amino acid sequence as shown in SEQ ID No: 1;
(b) a nucleotide sequence hybridsed with the nucleotide sequence described in (a) under strict conditions; and
(c) a nucleotide sequence having at least 70%, 75%, 80%, 85%, 90%, 95% or 97% of consistency comparing to the nucleotide sequence as described in (a).

9. A pharmaceutical combination for treatment of cancer, comprising a chemotheraputic agent in an amount for effective treatment, the chemotherapeutic agent being selected from antibiotic agents, anti-metabolic agents or any combination thereof, and a PDCD5 polypeptide in an amount of effective sensitization, wherein the PDCD5 polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).

10. The pharmaceutical combination according to claim 9, wherein the PDCD5 polypeptide is coded by a polynucleotide of a nucleotide sequence selected from the group consisting of:
(a) a nucleotide sequence coding an amino acid sequence as shown in SEQ ID No: 1;
(b) a nucleotide sequence hybridsed with a nucleotide sequence described in (a) under strict conditions; and
(c) a nucleotide sequence having at least 70%, 75%, 80%, 85%, 90%, 95% or 97% of consistency comparing to the nucleotide sequence as described in (a).

11. A method for treatment of cancer, such as liver cancer, cervical cancer, and leukemia, of a subject, the method comprising applying additional PDCD5 polypeptide or polynucleotide that codes the polypeptide in an amount for effective treatment prior to, after or during the period of applying a chemotherapeutic agent to the subject, wherein the PDCD5 polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).

12. The method according to claim 11, wherein the polynucleotide comprises a nucleotide sequence seclected from the group consisting of:
(a) a nucleotide sequence coding the PDCD5 polypeptide of (1), (2) or (3);
(b) a nucleotide sequence hybridsed with the nucleotide sequence described in (a) under strict conditions; and
(c) a nucleotide sequence having at least 70%, 75%, 80%, 85%, 90%, 95% or 97% of consistency comparing to the nucleotide sequence as described in (a).

13. The method according to claim 12, wherein the chemotherapeutic agent is selected from antibiotic agents, antimetabolic agents; preferably selected from neucleic acid synthesis interferring agents, DNA intercalating agents that interfere with RNA transcription, and mitochondria effecting agents; most preferrably selected from daunorubicin, idarubicin, doxorubicin, cytarabine, arsenic acids, and any combination thereof.

14. The method according to claim 12, wherein the polynucleotide is an expression vector such as in a form of a nucleic acid vaccine.

15. A method to reduce from damages or further damages of normal or diseased tissue or organ of a patient needing treatment, preferably the damages being those caused by trauma and/or occurred inside of body or induced by foreign substances, such as a pharmaceutical agent, applied from outside of body, more preferably the foreign substances being selected from alcohols, heavy metal salts, toxic metabolites generated from diseases, pharmaceutical agents especially chemotheropeutic agents, toxic substances contained in tobaccos, biological toxicants, chemical toxicants, and any combination thereof, the method comprising applying to the subject PDCD5 polypeptide or polynucleotide that codes the polypeptide in an amount for effective treatment, wherein the PDCD5 polypeptide is selected from the group consisting of:
(1) a polypeptide having an amino acid sequence as shown in SEQ ID No: 1;
(2) a polypeptide having an amino acid sequence containing at least 70%, 75%, 80%, 85%, 90%, 95% or 97% of homology of the SEQ ID No: 1, and having substantially the same biological function or activity comparing to that of the polypeptide of (1); and
(3) a functional fragment, variant, analog or derivative of the polypeptide of (1) or (2), having substantially the same biological function or activity comparing to that of the polypeptide of (1) or (2).
